# EUROPEAN PATENT APPLICATION

(11) **EP 3 650 022 A1**
(43) Date of publication of application: **13.05.2020**
(21) Application number: 19200380.4
(22) Date of filing: 17.02.2011
(51) Int. Cl.: A61K 31/437, A61P 1/12, A61P 31/04

(54) **METHODS FOR TREATING RIFAMPIN RESISTANT CLOSTRIDIUM DIFFICILIS INFECTIONS**

(30) Priority: 18.02.2010 US 30583210 P
(62) Divisional of application: 11745200.3
(71) Applicant: Salix Pharmaceuticals, Ltd., Morrisville, NC 27560 (US); Board of Regents of the University of Texas System, Austin, TX 78701 (US)
(72) Inventor: DU PONT, Herbert L., Houston, TX 77030 (US); GOLDEN, Pam, Durham, NC 27713 (US); JIANG, Zhi-Dong, Missouri City, TX 77459 (US); DARKOH, Charles, Houston, TX 77025 (US); BROWN, Eric L., Houston, TX 77030 (US)
(74) Representative: Hiebl, Inge Elisabeth

(57) **Abstract**

The present invention provides new methods and kits for treating and preventing bacterial infection.

SEQUENCE LISTING

<110> SALIX PHARMACEUTICALS, LTD.
      BOARD OF REGENTS OF THE UNIVERSITY OF TEXAS SYSTEM

<120> METHODS FOR TREATING INFECTION

<130> 24534EP1

<140> EP 19200380.4
<141> 2011-02-17

<150> US61/305,832
<151> 2010-02-18

<160> 8

<170> PatentIn version 3.5

<210> 1
<211> 19
<212> DNA
<213> Artificial Sequence

<220>
<221> source
<223> /note="Description of Artificial Sequence: Synthetic
      primer"

<400> 1
tcaccttccc ctcaggatg                                                    19


<210> 2
<211> 20
<212> DNA
<213> Artificial Sequence

<220>
<221> source
<223> /note="Description of Artificial Sequence: Synthetic
      primer"

<400> 2
tacaagcagg attacaacac                                                   20


<210> 3
<211> 19
<212> DNA
<213> Artificial Sequence

<220>
<221> source
<223> /note="Description of Artificial Sequence: Synthetic
      primer"

<400> 3
acggcgttac tatcctctc                                                    19


<210> 4
<211> 20

<212> DNA
<213> Artificial Sequence

<220>
<221> source
<223> /note="Description of Artificial Sequence: Synthetic
      primer"

<400> 4
tggtctcggt cagatatgtg                                                   20


<210> 5
<211> 20
<212> DNA
<213> Artificial Sequence

<220>
<221> source
<223> /note="Description of Artificial Sequence: Synthetic
      primer"

<400> 5
agccacactg gaactgagac                                                   20


<210> 6
<211> 19
<212> DNA
<213> Artificial Sequence

<220>
<221> source
<223> /note="Description of Artificial Sequence: Synthetic
      primer"

<400> 6
agccatttca ccgctacac                                                    19


<210> 7
<211> 21
<212> DNA
<213> Artificial Sequence

<220>
<221> source
<223> /note="Description of Artificial Sequence: Synthetic
      primer"

<400> 7
ctttgagggg caggttttcg t                                                 21


<210> 8
<211> 20
<212> DNA
<213> Artificial Sequence

<220>
<221> source
<223> /note="Description of Artificial Sequence: Synthetic
      primer"

<400> 8
cttagagaga ggccgtcgga 20

## Description

### RELATED APPLICATIONS

This application claims the benefit of US Provisional Application No.: 61/305,832, filed February 18, 2010, the entire contents of which are expressly incorporated herein by reference.

### BACKGROUND

Rifaximin (INN; see The Merck Index, XIII Ed., 8304) is an antibiotic belonging to the rifamycin class of antibiotics, e.g., a pyrido-imidazo rifamycin. Rifaximin exerts its broad antibacterial activity, for example, in the gastrointestinal tract against localized gastrointestinal bacteria that cause infectious diarrhea, irritable bowel syndrome, small intestinal bacterial overgrowth, Crohn's disease, and/or pancreatic insufficiency. It has been reported that rifaximin is characterized by a negligible systemic absorption, due to its chemical and physical characteristics (Descombe J.J. et al. Pharmacokinetic study of rifaximin after oral administration in healthy volunteers. Int J Clin Pharmacol Res, 14 (2), 51-56, (1994*)).*

Travelers' diarrhea is a common illness in travelers from industrialized countries to developing nations. It is commonly caused by virulence factors expressed by intestinal bacterial pathogens (eg., ETEC, EAEC, and *S sonnei*) that either directly damage surrounding tissue or elicit an immune response. In addition to Travelers' Diarrhea, a number of other disorders are characterized by bacterial infection of the gastrointestinal tract.

Accordingly, a need exists to for compositions and methods to effectively treat bacterial infections.

### SUMMARY

Disclosed herein are methods of preventing, ameliorating and/or bacterial infection. In general, subjects who may benefit from treatment with a rifamycin class antibiotic (e.g., rifaximin) include those who are susceptible to or have a bacterial infection. Exemplary infections include those bacterial infections that cause traveler's diarrhea.

Accordingly, in one aspect, the invention provides methods of treating a C. difficle infection (CDI) in a subject, by administering rifaximin to a subject, thereby treating CDI.

In one embodiment, the CDI is resistant to vancomycin and/or metronidazole and/or rifampin.

In one embodiment, the CDI is hospital acquired. In another embodiment, the CDI is caused by a hypervirulant strain of C. difficle. In one embodiment, the hypervirulant strain comprises genes encoding one or more of Toxin A, Toxin B, and Binary Toxin. In a specific embodiment, the hypervirulant strain comprises a deletion in the *tdcC* gene.

In another aspect, the instant invention provides methods of altering the virulence of bacteria causing Traveler's Diarrhea (TD) in a subject, by administering rifaximin to a subject having TD, thereby altering the virulence of the bacteria causing TD.

In one embodiment, the virulence of the bacteria is decreased, e.g., the virulence is decreased by decreasing the expression of one or more virulence factors. In one embodiment, the one or more virulence factors are selected from the group consisting of heat-stable enterotoxin (ST) and heat-labile enterotoxin (LT). In another embodiment, the virulence is decreased by decreasing the expression of one or more surface adhesion factors, e.g., CS2/CS3 and/or CS6.

In another embodiment, the virulence is decreased by decreasing the expression of one or more endopeptidases, e.g., matrix metalloproteases such as MMP-9.

In one embodiment, the rifaximin is administered as sub-bactericidal concentrations.

In another embodiment, the bacteria is E. coli, e.g., enterotoxigenic E. coli (ETEC) or exteroaggregative E. Coli (EAEC). In another embodiment, the bacteria is B. anthracis.

In another aspect, the instant invention provides methods of decreasing the ability of bacteria to attach to epithelial calls by contacting the epithelial cells with rifaximin prior to exposure to the bacteria, thereby decreasing the ability of the bacteria to attach to the epithelial cells.

In one embodiment, the inflammatory cytokine release from the cell is inhibited.

In another embodiment, the bacteria is E. coli, e.g., enterotoxigenic E. coli (ETEC) or exteroaggregative E. Coli (EAEC). In another embodiment, the bacteria is B. anthracis.

In one embodiment, the bacteria cause Traveler's diarrhea.

In another aspect, the instant invention provides methods of preventing a disease or disorder characterized by bacterial adhesion to epithelial cells of a subject, by administering rifaximin to a subject prior to exposure to the bacteria, thereby preventing a disease or disorder characterized by bacterial adhesion to epithelial calls.

In another embodiment, the bacteria is E. coli, e.g., enterotoxigenic E. coli (ETEC) or exteroaggregative E. Coli (EAEC). In another embodiment, the bacteria is B. anthracis.

In one aspect, presented herein are methods of treating or preventing bacterial infection, comprising: providing a container comprising rifaximin, wherein the container comprises printed labeling which describes administration instructions; and administering rifaximin from the container to the subject.

In one aspect, the rifaximin is Form α, Form β, Form γ, Form δ, Form ε, Form ζ, Form η, Form α-dry, Form ι, Form β-1, Form β-2, Form ε-dry, mesylate Form or amorphous Forms of rifaximin and a pharmaceutically acceptable carrier. The rifaximin may be formulated as a pharmaceutical composition.

In one embodiment, the pharmaceutical composition further comprises excipients.

According to another embodiment, the excipients comprise one or more of a diluting agent, binding agent, lubricating agent, disintegrating agent, coloring agent, flavorings agent or sweetening agent.

In another embodiment, the composition is formulated for selected coated and uncoated tablets, hard and soft gelatin capsules, sugar-coated pills, lozenges, wafer sheets, pellets and powders in sealed packet. In one embodiment, the composition is formulated for topical use.

In one embodiment, rifaximin is administered to a subject for 14 days.

In one embodiment, removing comprises instructing a subject by following dosing instructions on a package insert of a pharmaceutical product.

In one embodiment, the package insert instructs to administer the rifaximin for 14 days.

In one embodiment, the product comprises 550mg of rifaximin labeled for treatment of, for example, Travelers' diarrhea.

In one embodiment, the product comprises 550mg of rifaximin.

In one embodiment, a package insert of a pharmaceutical product warns of adverse events, including, for example, infections and infestations, gastrointestinal disorders, nervous system disorders, and musculoskeletal and connective tissue disorders.

Other embodiments of the invention are disclosed *infra.*

Moreover, the invention comprises the following embodiments:
1. A method of treating a C. difficle infection (CDI) in a subject, comprising:
   administering rifaximin to a subject,
   thereby treating CDI.
2. The method of item 1, wherein the CDI is resistant to vancomycin.
3. The method of item 1, wherein the CDI is resistant to metronidazole.
4. The method of item 1, wherein the CDI is resistant to rifampin.
5. The method of item 1, wherein the CDI is hospital acquired.
6. The method of item 1, wherein the CDI is a caused by a hypervirulant strain of C. difficle.
7. The method of item 6, wherein the hypervirulant strain comprises genes encoding one or more of Toxin A, Toxin B, and Binary Toxin.
8. The method of item 7, wherein the hypervirulant strain comprises a deletion in the *tdcC* gene.
9. A method of altering the virulence of bacteria causing Traveler's Diarrhea (TD) in a subject, comprising:
   administering rifaximin to a subject having TD;
   thereby altering the virulence of the bacteria causing TD.
10. The method of item 9, wherein the virulence of the bacteria is decreased.
11. The method of item 10, wherein the virulence is decreased by decreasing the expression of one or more virulence factors.
12. The method of item 11, wherein the one or more virulence factors are selected from the group consisting of heat-stable enterotoxin (ST) and heat-labile enterotoxin (LT).
13. The method of item 10, wherein the virulence is decreased by decreasing the expression of one or more surface adhesion factors.
14. The method of item 13, wherein the one or more surface adhesion factors are selected from the group consisting of CS2/CS3 and CS6.
15. The method of item 10, wherein the virulence is decreased by decreasing the expression of one or more endopeptidases.
16. The method of item 15, wherein the one or more endopeptidases are matrix metalloproteases.
17. The method of item 16, wherein one matrix metalloprotease is MMP-9.
18. The method of item 9, wherein the rifaximin is administered as sub-bactericidal concentrations.
19. The method of item 9, wherein the bacteria is E. coli.
20. The method of item 19, wherein the E. coli is enterotoxigenic E. coli (ETEC) or exteroaggregative E. Coli (EAEC).
21. The method of item 9, wherein the bacteria is B. anthracis.
22. A method for decreasing the ability of bacteria to attach to epithelial calls comprising:
   contacting the epithelial cells with rifaximin prior to exposure to the bacteria,
   thereby decreasing the ability of the bacteria to attach to the epithelial cells.
23. The method of item 22, wherein inflammatory cytokine release from the cell is inhibited.
24. The method of item 22, wherein the bacteria is E. coli.
25. The method of item 24, wherein the E. coli is enterotoxigenic E. coli (ETEC) or exteroaggregative E. Coli (EAEC).
26. The method of item 22, wherein the bacteria is B. anthracis.
27. The method of item 22, wherein the bacteria cause Traveler's diarrhea.
28. A method of preventing a disease or disorder characterized by bacterial adhesion to epithelial cells of a subject, comprising:
   administering rifaximin to a subject prior to exposure to the bacteria,
   thereby preventing a disease or disorder characterized by bacterial adhesion to epithelial calls.
29. The method of item 28, wherein the bacteria is E. coli.
30. The method of item 29, wherein the E. coli is enterotoxigenic E. coli (ETEC) or exteroaggregative E. Coli (EAEC).
31. The method of item 28, wherein the bacteria is B. anthracis.

### BRIEF DESCRIPTION OF THE DRAWINGS

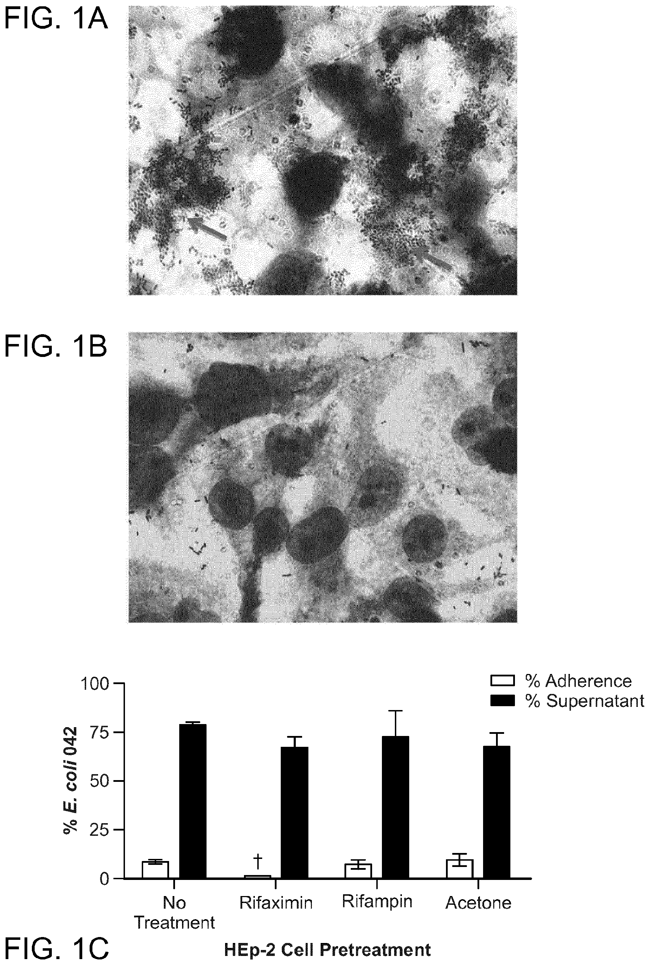

Figure 1 depicts EAEC attachment following pretreatment with rifaximin. (A) Untreated HEp-2 cells incubated with 7 x 10⁶ EAEC bacteria. The arrows indicate EAEC attaching to the HEp-2 cell and slide surfaces (magnification, x 100). (B) HEp-2 cells pretreated for 24 h with rifaximin (32 ug/ml), washed, and then incubated with 7 x 10⁶ EAEC bacteria (magnification, x100). The arrow indicates cells attaching to the slide surface and leading edge of HEp-2 cells. (C) Concentrations of EAEC in the supernatant or attached to the cell surface. HEp-2 cells grown to confluence in 24-well plates were pretreated with rifaximin, acetone, or rifampin, or were left untreated, for 24 h; then they were washed as described in Materials and Methods and were incubated with EAEC for 3 h in triplicate. To determine the CFU of EAEC in the supernatant, supernatants from respective wells were collected, and the wells were washed three times with PBS (200 ul each time). The supernatants and the washes were combined, centrifuged, resuspended in 1 ml PBS, serially diluted, and plated onto LB agar plates. To determine the number of EAEC bacteria attached, HEp-2 cells were trypsinized, washed, and plated onto LB plates as described above. The data are expressed as mean percentages of the total bacteria added to the wells that were found in the supernatant or attached to the cell surface. Error bars, SE. CFU counts in the supernatants or cell fractions of triplicate wells demonstrated reduced EAEC adherence to HEp-2 cells pretreated with rifaximin. †, *P* _ 0.05 by an unpaired Student *t* test.

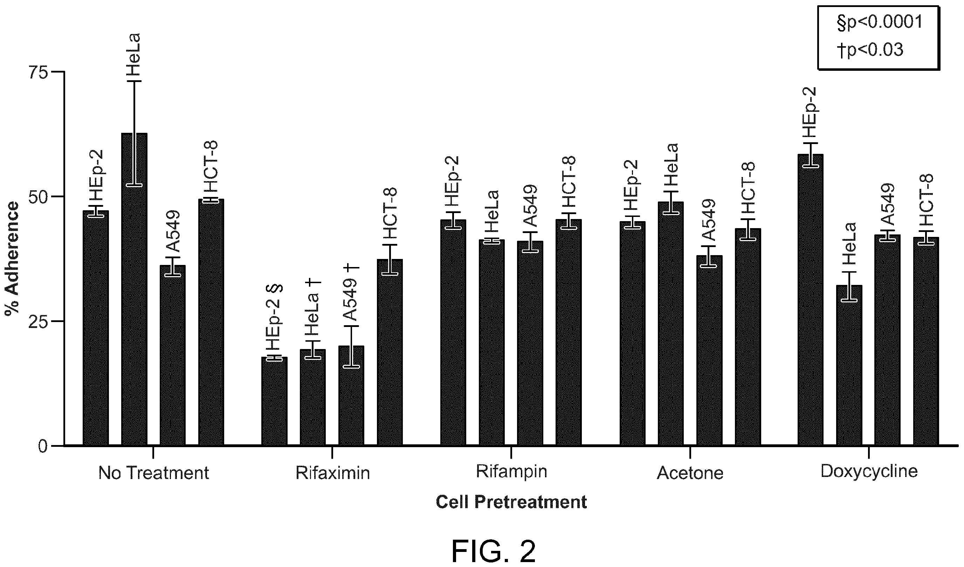

Figure 2 depicts rifaximin pretreatment reduced EAEC binding to epithelial cells. Confluent HEp-2, HeLa, A549, or HCT-8 cells were cultured for 24 h either in medium only or in medium containing 64 µg/ml of either rifaximin, rifampin, acetone (64 µl), or doxycycline in triplicate. The wells were washed, and 1 x 107 EAEC bacteria were added and incubated for 3 h. Then the wells were washed three times with 1 ml of PBS (each wash), and the cells were lysed by addition of 1 ml of sterile distilled water. Serial dilutions were then made in sterile PBS and plated onto LB agar plates to determine the number of CFU per well. The data are expressed as the percentage of the total number of bacteria added to the wells that adhered to the cells. P values, determined by an unpaired Student t test, were_0.0001 (†) and _0.03 (§).

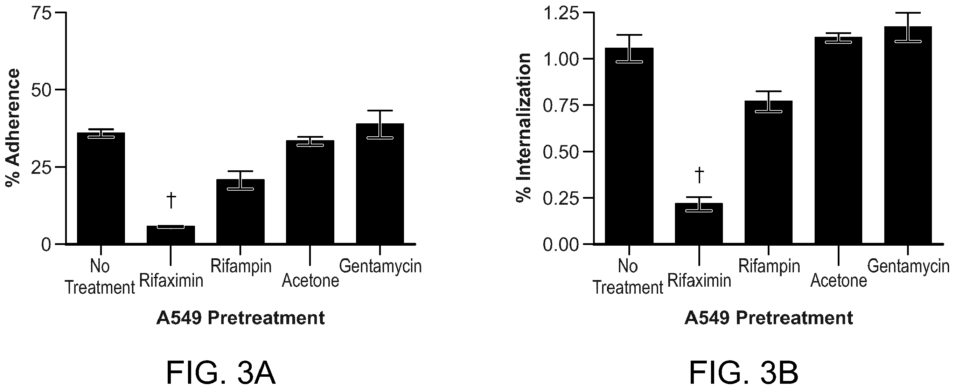

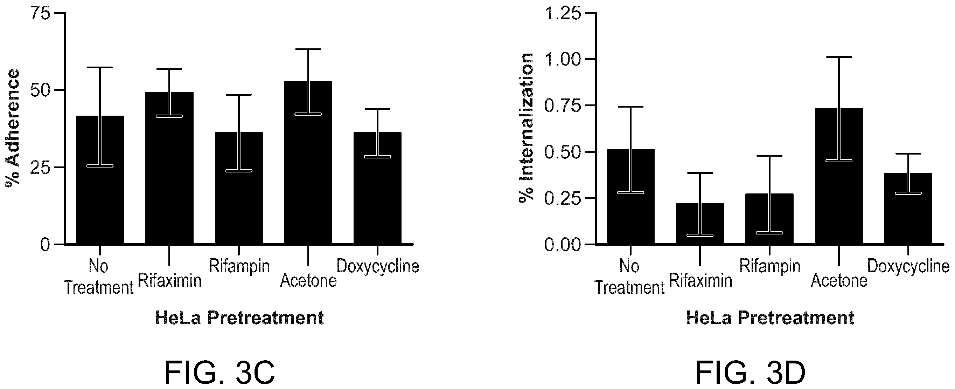

Figure 3 depicts the effects of rifaximin pretreatment on the adherence and internalization of *B. anthracis* and *S. sonnei.* Graphs show the adherence (A and C) or internalization (B and D) of *B. anthracis* (A and B) or *S. sonnei* (C and D). *B. anthracis* spores (4.8 x 10⁵) or *S. sonnei* bacteria (1 x 10⁷) were cocultured in wells of 24-well plates containing either A549 or HeLa cells, respectively. For adherence assays, the respective bacteria were incubated for 1 h; the wells were washed; and the cells were plated onto LB agar plates. For internalization, an additional 1-h incubation with gentamicin (100 µg/ml) was carried out to kill any noninternalized bacteria. The wells were then washed, and the cells were plated onto LB agar plates. The data are expressed as the mean percentages of the total cells added that adhered or were internalized. Error bars, SE. The experiment was repeated twice and with similar results. †, *P* _ 0.002 by an unpaired Student t test.

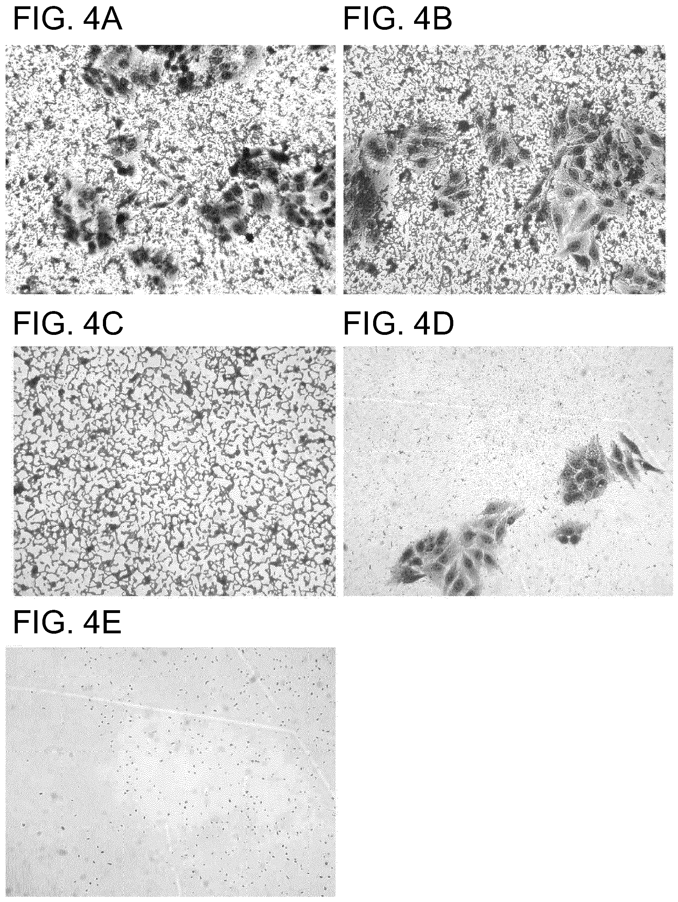

Figure 4 depicts the effects of dialyzed supernatants on EAEC adherence. EAEC adherence was assessed by adding 750 µl of an EAEC solution (7x106 bacteria/ml) mixed with 750_1 of either dialyzed conditioned medium only (A and C), dialyzed rifampin-conditioned medium (B), or dialyzed rifaximin-conditioned medium (D and E). EAEC adherence was monitored in the presence (A, B, and D) or absence (C and E) of HEp-2 cells. After a 3-h incubation, the chambers were washed and examined microscopically (magnification, _40) following Wright-Giemsa staining. These experiments demonstrated that a factor (or the lack thereof) in the supernatant of rifaximin-treated cells affected EAEC adherence. This experiment was repeated twice with similar results.

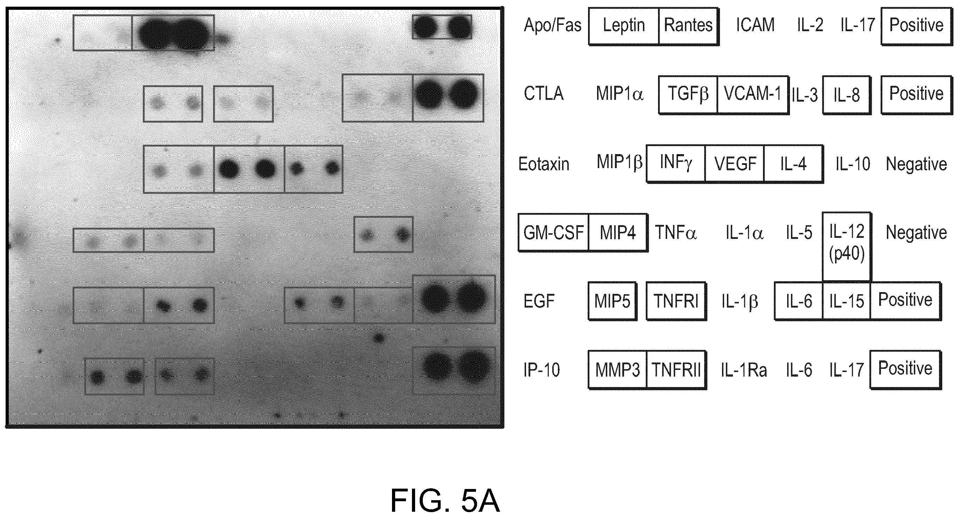

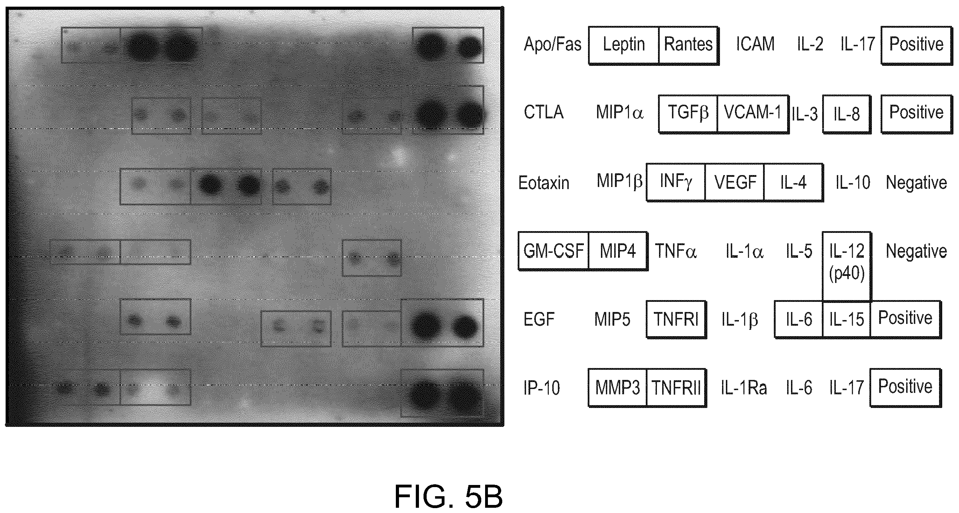

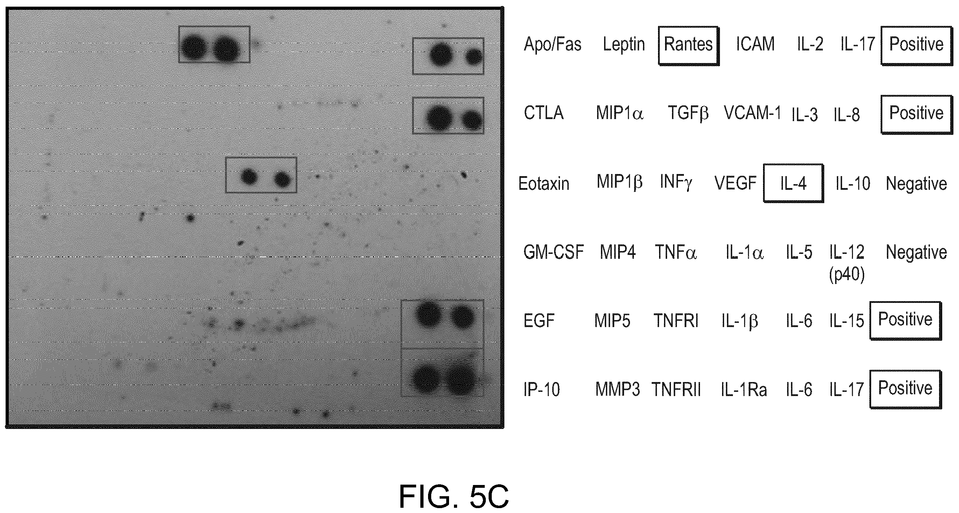

Figure 5 depicts the supernatant cytokine profile analysis. Supernatants from HEp-2 cells cultured for 24 h without antibiotics (A), in the presence of rifampin (B), or in the presence of rifaximin (C) were analyzed using a Panomics cytokine array. Each membrane array is designed to detect as many as 36 different cytokines (indicated to the right) in duplicate, together with positive and negative internal controls. The blots shown were incubated with the respective supernatants at the same time and were developed on the same film simultaneously. Supernatants from HEp-2 cells treated with doxycycline or acetone were also analyzed. This experiment was carried out three times with similar results, demonstrating that rifaximin reduced the expression of various proinflammatory cytokines.

### DETAILED DESCRIPTION

Rifaximin (USAN, INN; see The Merck Index, XIII Ed., 8304, CAS No. 80621-81-4), (2S,16Z,18E,20S,21S,22R, 23R,24R,25S,26S,27S,28E)-5,6,21,23,25 Pentahydroxy-27-methoxy-2,4,11,16,20,22,24,26-octamethyl-2,7-(epoxypentadeca-(1,11,13)trienimino)benzofuro(4,5-e)pyrido(1,2,-a)benzimidazole-1,15(2H)-dione,25-acetate), is a semi-synthetic antibiotic produced from rifamycin O. Rifaximin is a molecule belonging to the rifamycin class of antibiotics, e.g., a pyrido-imidazo rifamycin. Rifaximin exerts a broad antibacterial activity, for example, in the gastrointestinal tract against localized gastrointestinal bacteria that cause infectious diarrhea, irritable bowel syndrome, small intestinal bacterial overgrowth, Crohn's disease, and/or pancreatic insufficiency.

Rifaximin is also described in Italian Patent IT 1154655 and EP 0161534. EP patent 0161534 discloses a process for rifaximin production using rifamycin O as the starting material (The Merck Index, XIII Ed., 8301). US 7,045,620 B1 discloses polymorphic forms of rifaximin, as do USSN 11/658,702; USSN 61/031,329; USSN 12/119,622; USSN 12/119,630; USSN 12/119,612; USSN 12/119,600; USSN 11/873,841; Publication WO 2006/094662; and USSN 12/393012. The applications and patents referred to here are incorporated herein by reference in their entirety for all purposes.

A rifamycin class antibiotic is, for example, a compound having the structure of Formula I: 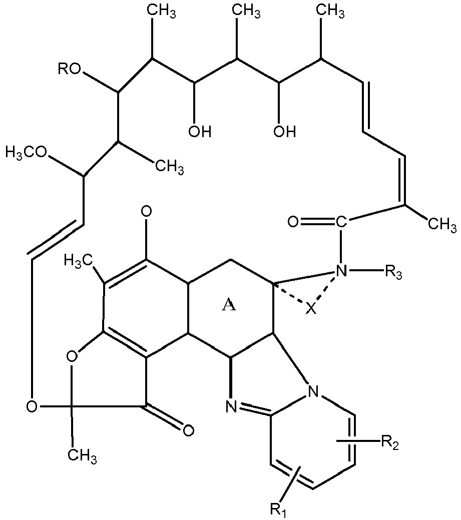 wherein A may be the structure A₁: 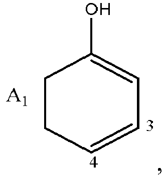 or the structure A₂ 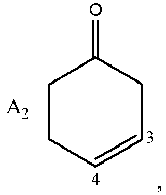 wherein, -x- is a covalent chemical bond or nil; R is hydrogen or acetyl;
R₁ and R₂ independently represent hydrogen, (C₁₋₄)alkyl, benzyloxy, mono- and di-(C₁₋₃)alkylamino-(C₁₋₄)alkyl, (C₁₋₃)alkoxy-(C₁₋₄)alkyl, hydroxymethyl, hydroxy-(C₂₋₄)-alkyl, nitro or R₁ and R₂ taken together with two consecutive carbon atoms of the pyridine nucleus form a benzene ring unsubstituted or substituted by one or two methyl or ethyl groups; R₃ is a hydrogen atom or nil; with the proviso that, when A is A₁, -x- is nil and R₃ is a hydrogen atom; with the further proviso that, when A is A₂, -x- is a covalent chemical bond and R₃ is nil.

Also described herein is a compound as defined above, wherein A is A₁ or A₂ as above indicated, -x- is a covalent chemical bond or nil, R is hydrogen or acetyl, R₁ and R₂ independently represent hydrogen, (C₁₋₄)alkyl, benzyloxy, hydroxy-(C₂₋₄) alkyl, di-(C₁₋₃) alkylamino-(C₁₋₄) alkyl, nitro or R₁ and R₂ taken together with two consecutive carbon atoms of the pyridine nucleus form a benzene ring and R₃ is a hydrogen atom or nil; with the proviso that, when A is A₁, -x- is nil and R₃ is a hydrogen atom; with the further proviso that, when A is A₂, -x- is a covalent chemical bond and R₃ is nil.

Also described herein is a compound as defined above, wherein A is A₁ or A₂ as above indicated, -x- is a covalent chemical bond or nil, R is acetyl, R₁ and R₂ independently represent hydrogen, (C₁₋₄) alkyl or R₁ and R₂ taken together with two consecutive carbon atoms of the pyridine nucleus form a benzene ring and R₃ is a hydrogen atom or nil; with the proviso that, when A is A₁, -x- is nil and R₃ is a hydrogen atom; with the further proviso that, when A is A₂, -x- is a covalent chemical bond and R₃ is nil.

Also described herein is a compound as defined above, which is 4-deoxy-4'-methyl-pyrido[1',2'-1,2]imidazo [5,4-c]rifamycin SV. Also described herein is a compound as defined above, which is 4-deoxy-pyrido [1',2':1,2]imidazo [5,4-c] rifamycin SV.

Also described herein is a compound as defined above, wherein A is as described above,-x- is a covalent chemical bond or nil; R is hydrogen or acetyl; R₁ and R₂ independently represent hydrogen, (C₁₋₄) alkyl, benzyloxy, mono- and di-(C₁₋₃)alkylamino(C₁₋₄)alkyl, (C₁₋₃)alkoxy-(C₁₋₄)alkyl, hydroxymethyl, hydroxy-(C₂₋₄)-alkyl, nitro or R₁ and R₂ taken together with two consecutive carbon atoms of the pyridine nucleus form a benzene ring unsubstituted or substituted by one or two methyl or ethyl groups; R₃ is a hydrogen atom or nil; with the proviso that, when A is A₁, -x- is nil and R₃ is a hydrogen atom; with the further proviso that, when A is A₂, -x- is a covalent chemical bond and R₃ is nil.

Rifaximin is a compound having the structure of formula II:

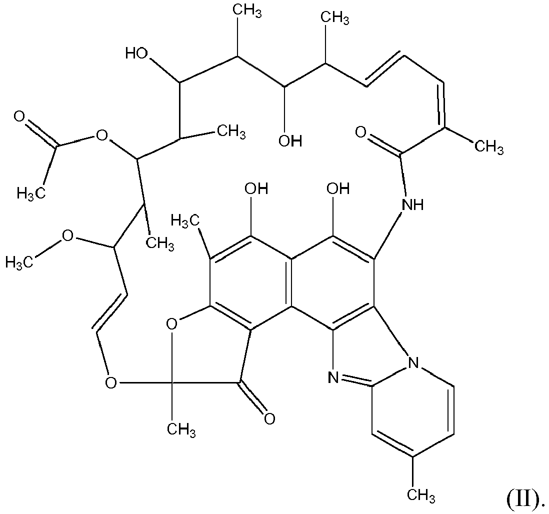

In certain embodiments, the antibiotic comprises one or more of a rifamycin, aminoglycoside, amphenicol, ansamycin, β-Lactam, carbapenem, cephalosporin, cephamycin, monobactam, oxacephem, lincosamide, macrolide, polypeptide, tetracycline, or a 2,4-diaminopyrimidine class antibiotic. Exemplary antibiotics of these classes are listed below.

Without wishing to be bound by any particular scientific theories, rifaximin acts by binding to the beta-subunit of the bacterial deoxyribonucleic acid-dependent ribonucleic acid (RNA) polymerase, resulting in inhibition of bacterial RNA synthesis. It is active against numerous gram (+) and (-) bacteria, both aerobic and anaerobic. *In vitro* data indicate rifaximin is active against species of *Staphylococcus, Streptococcus, Enterococcus,* and *Enterobacteriaceae.*

"Rifaximin", as used herein, includes solvates and polymorphous forms of the molecule, including, for example, Form α, Form β, Form γ Form δ, Form ε, Form ζ, Form η, Form α-dry, Form ι, Form β-1, Form β-2, Form ε-dry, mesylate Form or amorphous Forms of rifaximin. These forms are described in more detail, for example, in USSN 11/873,841; USSN 11/658,702; EP 05 004 635.2, filed 03 May 2005; USPN 7,045,620; US 61/031,329; and G. C. Viscomi, et al., CrystEngComm, 2008, 10, 1074-1081 (April 2008). Each of these references is hereby incorporated by reference in entirety.

Medicinal preparations may contain gastrointestinal specific antibiotics together with usual excipients, discussed infra.

"Polymorphism", as used herein, refers to the occurrence of different crystalline forms of a single compound in distinct hydrate status, e.g., a property of some compounds and complexes. Thus, polymorphs are distinct solids sharing the same molecular formula, yet each polymorph may have distinct physical properties. Therefore, a single compound may give rise to a variety of polymorphic forms where each form has different and distinct physical properties, such as solubility profiles, melting point temperatures, hygroscopicity, particle shape, density, flowability, compatibility and/or x-ray diffraction peaks. The solubility of each polymorph may vary, thus, identifying the existence of pharmaceutical polymorphs is essential for providing pharmaceuticals with predictable solubility profiles. It is desirable to investigate all solid state forms of a drug, including all polymorphic forms, and to determine the stability, dissolution and flow properties of each polymorphic form. Polymorphic forms of a compound can be distinguished in a laboratory by X-ray diffraction spectroscopy and by other methods such as, infrared spectrometry. For a general review of polymorphs and the pharmaceutical applications of polymorphs see G. M. Wall, Pharm Manuf. 3, 33 (1986); J. K. Haleblian and W. McCrone, J Pharm. Sci., 58, 911 (1969); and J. K. Haleblian, J. Pharm. Sci., 64, 1269 (1975), all of which are incorporated herein by reference. As used herein, the term polymorph is occasionally used as a general term in reference to the forms of rifaximin and includes within the context, salt, hydrate, polymorphic and amorphous forms of rifaximin as disclosed herein. This use depends on context and will be clear to one of skill in the art.

"GI specific antibiotic," and "GI antibiotic" as used herein include antibiotic known to have an effect on GI disease. For example, a rifamycin class antibiotic (e.g., rifaximin), neomycin, metronidazole, teicoplanin, ciprofloxacin, doxycycline, tetracycline, augmentin, cephalexin, penicillin, ampicillin, kanamycin, rifamycin, vancomycin, and combinations thereof are useful GI specific antibiotics. Even more preferable are GI specific antibiotics with low systemic absorption, for example, rifaximin. Low systemic absorption includes, for example, less than 10% absorption, less than 5% absorption, less than 1% absorption and less than 0.5% absorption. Low systemic absorption also includes, for example, from between about 0.01-1% absorption, from between about 0.05 -1% absorption, from between about 0.1-1% absorption, from between about 1-10% absorption, or from between about 5 - 20% absorption.

"Ameliorate," "amelioration," "improvement" or the like refers to, for example, a detectable improvement or a detectable change consistent with improvement that occurs in a subject or in at least a minority of subjects, e.g., in at least about 2%, 5%, 10%, 15%, 20%, 25%, 30%, 40%, 50%, 60%, 70%, 75%, 80%, 85%, 90%, 95%, 98%, 100% or in a range between about any two of these values. Such improvement or change may be observed in treated subjects as compared to subjects not treated with rifaximin, where the untreated subjects have, or are subject to developing, the same or similar disease, condition, symptom or the like. Amelioration of a disease, condition, symptom or assay parameter may be determined subjectively or objectively, e.g., self assessment by a subject(s), by a clinician's assessment or by conducting an appropriate assay or measurement, including, e.g., a quality of life assessment, a slowed progression of a disease(s) or condition(s), a reduced severity of a disease(s) or condition(s), or a suitable assay(s) for the level or activity(ies) of a biomolecule(s), cell(s) or by detection of BD episodes in a subject. Amelioration may be transient, prolonged or permanent or it may be variable at relevant times during or after a GI specific antibiotic is administered to a subject or is used in an assay or other method described herein or a cited reference, e.g., within timeframes described infra, or about 1 hour after the administration or use of a GI specific antibiotic to about 7 days, 2 weeks, 28 days, or 1, 3, 6, 9 months or more after a subject(s) has received such treatment.

The "modulation" of, e.g., a symptom, level or biological activity of a molecule, or the like, refers, for example, that the symptom or activity, or the like is detectably increased or decreased. Such increase or decrease may be observed in treated subjects as compared to subjects not treated with a GI specific antibiotic, where the untreated subjects have, or are subject to developing, the same or similar disease, condition, symptom or the like. Such increases or decreases may be at least about 2%, 5%, 10%, 15%, 20%, 25%, 30%, 40%, 50%, 60%, 70%, 75%, 80%, 85%, 90%, 95%, 98%, 100%, 150%, 200%, 250%, 300%, 400%, 500%, 1000% or more or within any range between any two of these values. Modulation may be determined subjectively or objectively, e.g., by the subject's self assessment, by a clinician's assessment or by conducting an appropriate assay or measurement, including, e.g., quality of life assessments or suitable assays for the level or activity of molecules within a subject. Modulation may be transient, prolonged or permanent or it may be variable at relevant times during or after a GI specific antibiotic is administered to a subject or is used in an assay or other method described herein or a cited reference, e.g., within times descried infra, or about 1 hour of the administration or use of a GI specific antibiotic to about 2 weeks, 28 days, 3, 6, 9 months or more after a subject(s) has received a GI specific antibiotic.

The term "modulate" may also refer to increases or decreases in the activity of a cell in response to exposure to a GI specific antibiotic, e.g., the inhibition of proliferation and/or induction of differentiation of at least a sub-population of cells in an animal such that a desired end result is achieved, e.g., a therapeutic result of GI specific antibiotic used for treatment may increase or decrease over the course of a particular treatment.

The language "a prophylactically effective amount" of a compound refers to an amount of a compound of the invention of formula I, formula II, or otherwise described herein which is effective, upon single or multiple dose administration to the subject, in preventing or treating a bacterial infection.

As used herein, "subject" includes organisms which are capable of suffering from a bowel disease or other disorder treatable by a rifamycin class antibiotic (e.g., rifaximin) or who could otherwise benefit from the administration of a rifamycin class antibiotic (e.g., rifaximin) as described herein, such as human and non-human animals. Preferred human animals include human subjects. The term "non-human animals" of the invention includes all vertebrates, e.g., mammals, e.g., rodents, e.g., mice, and non-mammals, such as non-human primates, e.g., sheep, dog, cow, chickens, amphibians, reptiles, etc. At risk for a bacterial infection is meant to include a subject at risk of developing an infection or a person who is in remission from an infection or a person who may relapse, e.g., a subject suffering from immune suppression, a subject that has been exposed to a bacterial infection, physicians, nurses, a subject traveling to remote areas known to harbor bacteria that cause travelers' diarrhea, an aging person, a person with liver damage, etc.

The language "a prophylactically effective amount" of a compound refers to an amount of a compound of the invention of formula I, formula II, or otherwise described herein which is effective, upon single or multiple dose administration to the subject, in preventing or treating an infection or the disease or disorder caused by the infection.

The term "administration" or "administering" includes routes of introducing a GI specific antibiotic to a subject to perform their intended function. Examples of routes of administration that may be used include injection, oral, inhalation, vaginal, rectal and transdermal. The pharmaceutical preparations may be given by forms suitable for each administration route. For example, these preparations are administered in tablets or capsule form, by injection, inhalation, eye lotion, eye drops, ointment, suppository, etc. administration by injection, infusion or inhalation; topical by lotion or ointment; and rectal by suppositories. Oral administration is preferred. The injection can be bolus or can be continuous infusion. Depending on the route of administration, a GI specific antibiotic can be coated with or disposed in a selected material to protect it from natural conditions that may detrimentally effect its ability to perform its intended function. A GI specific antibiotic can be administered alone, or in conjunction with either another agent or agents as described above or with a pharmaceutically-acceptable carrier, or both. A GI specific antibiotic can be administered prior to the administration of the other agent, simultaneously with the agent, or after the administration of the agent. Furthermore, a GI specific antibiotic can also be administered in a proform, which is converted into its active metabolite, or more active metabolite *in vivo.*

Administration "in combination with" one or more further therapeutic agents includes simultaneous (concurrent) and consecutive administration in any order.

As will be readily apparent to one skilled in the art, the useful *in vivo* dosage to be administered and the particular mode of administration will vary depending upon the age, weight and mammalian species treated, the particular compounds employed, and/or the specific use for which these compounds are employed. The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved.

The term "obtaining" as in "obtaining a GI specific antibiotic" is intended to include purchasing, synthesizing or otherwise acquiring a GI specific antibiotic.

The language "a prophylactically effective amount" of a compound refers to an amount of a GI specific antibiotic which is effective, upon single or multiple dose administration to the subject, in preventing or treating an indication. In one embodiment, the indication is IBS.

The term "pharmaceutical agent composition" (or agent or drug) as used herein refers to a chemical compound, composition, agent or drug capable of inducing a desired therapeutic effect when properly administered to a patient. It does not necessarily require more than one type of ingredient.

As used herein, "durability of response" includes for example, adequate relief of symptoms after removal of treatment, continuous adequate relief of symptoms after removal of treatment, or response that is greater than or superior to placebo response. A response by a subject may be considered durable, for example, if they have a response to rifamycin after removal from treatment. The duration of response, may be, for example, 2 days, 7 days, two weeks, 3 weeks, 4 weeks, 12 weeks, between about 1 week and about 24 weeks or longer. The response may be measured, for example using one or more of the methods outlined below, including, for example, a subject's subjective assessment of their symptoms or a healthcare provider's or caretaker's assessment of a subject's symptoms.

As used herein, "selecting subject's who respond," "selection of subject's who respond" or the like, include, for example, determining that a subject has responded to treatment based on a decrease of infection symptoms and/or following label instructions to administer a product (e.g., rifaximin) for a certain period of time or the like. The determination or selection may be based on the label (e.g., package or package insert) instructions or on a subject's subjective assessment of their symptoms or a healthcare provider's or caretaker's assessment of a subject's symptoms.

### Methods of Treatment

Provided herein are methods of treating, preventing, or alleviating bacterial infection or the symptoms caused by bacterial infection comprising administering to a subject in need thereof an effective amount of rifaximin. Exemplary infections that can be treated or prevented using the methods of the invention include infections by E. coli, B. anthracis, or S. Sonnei.

In one embodiment, the recommendation (e.g., selection) is made on a label of a pharmaceutical product, which indicates that the rifaximin should be administered for 14 days (e.g., two weeks). For example, a subject at risk of developing a bacterial infection is administered rifaximin prior to exposure to the bacteria.

In another embodiment, the inventions provide methods for the treatment of C. difficle infection (CDI) in a subject, by administering rifaximin to the subject. In at least one embodiment, the CDI is resistant to one or more antibiotics, for example, vancomycin, metronidazole or rifampin.

In one embodiment, the CDI is a hospital acquired infection. In another embodiment, the CDI is a caused by a hypervirulant strain of C. difficle. In one embodiment, the hypervirulant strain comprises genes encoding one or more of Toxin A, Toxin B, and Binary Toxin. In another embodiment, the hypervirulant strain comprises a deletion in the *tdcC* gene.

Rifaximin may be used in various treatment regimes. These regimes may vary depending upon the subject and the type of treatment.

Rifaximin may be administered, for example, twice a day, three times a day, or four times or more often as necessary per day. Rifaximin may be administered in doses, for example of from about between 25 mg BID to about 3000 mg TID. Another example is administering rifaximin from between about 4.0 g/day to about 7.25 g/day. The rifaximin may be administered, for example, in tablet form, powered form, liquid for or in capsules.

Subjects in need thereof include subjects having or that are susceptible to bacterial infection, or are in remission from an infection.

As used herein, a therapeutically effective amount means an amount effective, when administered to a human or non-human subject, to provide a therapeutic benefit such as an amelioration of symptoms, e.g., an amount effective to decrease the symptoms of a bacterial infection, i.e., the symptoms of Traveler's Diarrhea.

In certain embodiments, the rifaximin is administered to a subject from between about 1 week to about 6 weeks in duration, from between about 8 weeks to about 12 weeks in duration, or from between 1 day to about 7 days. The rifaximin may be administered from between about 1 day and about 1 year, or from 1 week to about 24 weeks. The rifaximin may be administered intermittently or continuously during the course of treatment. Length of treatment may vary depending on the type and length of disease and the proper length of treatment may be easily determined by one of skill in the art having the benefit of this disclosure. Treatment may be prior to infection for a period of time suggested by a medical professional to reduce or eliminate the chance of bacterial infection.

For any of the embodiments, rifaximin may be administered, for example, once daily, twice daily, three times daily, or four times daily (or more often as necessary for a particular subject) to a subject.

Dosages, according to certain preferred embodiments, range from between about 50 to about 6000 mg of rifaximin administered daily. For example, a dose of 550 mg may be administered to a subject twice daily. Other appropriate dosages for methods according to this invention may be determined by health care professionals or by the subject. The amount of rifaximin administered daily may be increased or decreased based on the weight, age, health, sex or medical condition of the subject. One of skill in the art would be able to determine the proper dose for a subject based on this disclosure.

According to certain embodiments, rifaximin may be administered in combination with other compounds, including for example, chemotherapeutic agents, anti-inflammatory agents, anti-pyretic agents radiosensitizing agents, radioprotective agents, urologic agents, anti-emetic agents, and/or anti-diarrheal agents. for example, cisplatin, carboplatin, docetaxel, paclitaxel, flurouracil, capecitabine, gemcitabine, irinotecan, topotecan, etoposide, mitomycin, gefitinib, vincristine, vinblastine, doxorubicin, cyclophosphamide, celecoxib, rofecoxib, valdecoxib, ibuprofen, naproxen, ketoprofen, dexamethasone, prednisone, prednisolone, hydrocortisone, acetaminophen, misonidazole, amifostine, tamsulosin, phenazopyridine, ondansetron, granisetron, alosetron, palonosetron, promethazine, prochlorperazine, trimethobenzamide, aprepitant, diphenoxylate with atropine, and/or loperamide.

### Pharmaceutical Preparations

The invention also provides pharmaceutical compositions, comprising an effective amount of a rifamycin class antibiotic ((e.g., rifaximin or a rifaximin polymorph) described herein and a pharmaceutically acceptable carrier. In a further embodiment, the effective amount is effective to treat a bacterial infection, e.g., small intestinal bacterial overgrowth.

For examples of the use of rifaximin and formulations thereof to treat Travelers' diarrhea, see Infante RM, Ericsson CD, Zhi-Dong J, Ke S, Steffen R, Riopel L, Sack DA, DuPont, HL. Enteroaggregative Escherichia coli Diarrhea in Travelers: Response to Rifaximin Therapy. Clinical Gastroenterology and Hepatology. 2004;2:135-138; and Steffen R, M.D., Sack DA, M.D., Riopel L, Ph.D., Zhi-Dong J, Ph.D., Sturchler M, M.D., Ericsson CD, M.D., Lowe B, M.Phil., Waiyaki P, Ph.D., White M, Ph.D., DuPont HL, M.D. Therapy of Travelers' Diarrhea With Rifaximin on Various Continents. The American Journal of Gastroenterology. May 2003, Volume 98, Number 5, all of which are incorporated herein by reference in their entirety.

One embodiment includes pharmaceutical compositions comprising rifaximin or any polymorphic form thereof and a pharmaceutically acceptable carrier. That is, formulations may contain only one polymorph or may contain a mixture of more than one polymorph. Polymorph, in this context, refers to any physical form, hydrate, acid, salt or the like of rifaximin. Mixtures may be selected, for example on the basis of desired amounts of systemic adsorption, dissolution profile, desired location in the digestive tract to be treated, and the like. The pharmaceutical composition further comprises excipients, for example, one or more of a diluting agent, binding agent, lubricating agent, disintegrating agent, coloring agent, flavoring agent or sweetening agent. Compositions may be formulated for selected coated and uncoated tablets, hard and soft gelatin capsules, sugar-coated pills, lozenges, wafer sheets, pellets and powders in sealed packet. For example, compositions may be formulated for topical use, for example, ointments, pomades, creams, gels and lotions.

In an embodiment, the rifamycin class antibiotic (e.g., rifaximin) is administered to the subject using a pharmaceutically-acceptable formulation, e.g., a pharmaceutically-acceptable formulation that provides sustained delivery of the rifamycin class antibiotic (e.g., rifaximin) to a subject for at least 12 hours, 24 hours, 36 hours, 48 hours, one week, two weeks, three weeks, or four weeks after the pharmaceutically-acceptable formulation is administered to the subject.

In certain embodiments, these pharmaceutical compositions are suitable for topical or oral administration to a subject. In other embodiments, as described in detail below, the pharmaceutical compositions of the present invention may be specially formulated for administration in solid or liquid form, including those adapted for the following: (1) oral administration, for example, drenches (aqueous or non-aqueous solutions or suspensions), tablets, boluses, powders, granules, pastes; (2) parenteral administration, for example, by subcutaneous, intramuscular or intravenous injection as, for example, a sterile solution or suspension; (3) topical application, for example, as a cream, ointment or spray applied to the skin; (4) intravaginally or intrarectally, for example, as a pessary, cream or foam; or (5) aerosol, for example, as an aqueous aerosol, liposomal preparation or solid particles containing the compound.

The phrase "pharmaceutically acceptable" refers to those rifamycin class antibiotic (e.g., rifaximin) described herein, compositions containing such compounds, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" includes pharmaceutically-acceptable material, composition or vehicle, such as a liquid or solid filler, diluent, excipient, solvent or encapsulating material, involved in carrying or transporting the subject chemical from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Compositions containing a rifamycin class antibiotic (e.g., rifaximin) include those suitable for oral, nasal, topical (including buccal and sublingual), rectal, vaginal, aerosol and/or parenteral administration. The compositions may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred percent, this amount will range from about 1% to about 99 % of active ingredient, preferably from about 5 % to about 70 %, most preferably from about 10 % to about 30 %.

Liquid dosage forms for oral or rectal administration of the rifamycin class antibiotic (e.g., rifaximin) include pharmaceutically-acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

In addition to inert diluents, the oral compositions can include adjuvants such as wetting agents, emulsifying and suspending agents, sweetening, flavoring, coloring, perfuming and preservative agents.

Suspensions, in addition to the active rifamycin class antibiotic (e.g., rifaximin) may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Pharmaceutical compositions of the invention for rectal or vaginal administration may be presented as a suppository, which may be prepared by mixing one or more rifamycin class antibiotic (e.g., rifaximin) with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active agent. Compositions which are suitable for vaginal administration can include pessaries, tampons, creams, gels, pastes, foams or spray formulations containing such carriers as are known in the art to be appropriate.

Dosage forms for the topical or transdermal administration of a rifamycin class antibiotic (e.g., rifaximin) can include powders, sprays, ointments, pastes, creams, lotions, gels, solutions, patches and inhalants. The active rifamycin class antibiotic (e.g., rifaximin) may be mixed under sterile conditions with a pharmaceutically-acceptable carrier, and with any preservatives, buffers, or propellants which may be required.

The ointments, pastes, creams and gels may contain, in addition to rifamycin class antibiotic (e.g., rifaximin), excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a rifamycin class antibiotic (e.g., rifaximin), excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

The rifamycin class antibiotic (e.g., rifaximin) can be alternatively administered by aerosol. This is accomplished, for example, by preparing an aqueous aerosol, liposomal preparation or solid particles containing the compound. A non-aqueous (e.g., fluorocarbon propellant) suspension could be used. Sonic nebulizers are preferred because they minimize exposing the agent to shear, which can result in degradation of the compound.

Examples of suitable aqueous and non-aqueous carriers which may be employed in the pharmaceutical compositions can include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, to prolong the effect of a drug, it is desirable to alter the absorption of the drug. This may be accomplished by the use of a liquid suspension of crystalline, salt oramorphous material having poor water solubility. The rate of absorption of the drug may then depend on its rate of dissolution which, in turn, may depend on crystal size and crystalline form. Alternatively, delayed absorption of a drug form is accomplished by dissolving or suspending the drug in an oil vehicle.

When rifamycin class antibiotics (e.g., rifaximin) are administered as pharmaceuticals, to humans and animals, they can be given *per se* or as a pharmaceutical composition containing, for example, 0.1 to 99.5% (more preferably, 0.5 to 90%) of active ingredient in combination with a pharmaceutically-acceptable carrier.

Regardless of the route of administration selected, the rifamycin class antibiotic (e.g., rifaximin), which may be used in a suitable hydrated form, and/or the pharmaceutical compositions of the present invention, are formulated into pharmaceutically-acceptable dosage forms by conventional methods known to those of skill in the art.

Actual dosage levels and time course of administration of the active ingredients in the pharmaceutical compositions of the invention may be varied so as to obtain an amount of the active ingredient which is effective to achieve the desired therapeutic response for a particular patient, composition, and mode of administration, without being toxic to the patient. An exemplary dose range is from 25 to 3000 mg per day.

In combination therapy treatment, both the compounds of this invention and the other drug agent(s) are administered to mammals (e.g., humans, male or female) by conventional methods. The agents may be administered in a single dosage form or in separate dosage forms. Effective amounts of the other therapeutic agents are well known to those skilled in the art. However, it is well within the skilled artisan's purview to determine the other therapeutic agent's optimal effective-amount range. In one embodiment of the invention in which another therapeutic agent is administered to an animal, the effective amount of the compound of this invention is less than its effective amount in case the other therapeutic agent is not administered. In another embodiment, the effective amount of the conventional agent is less than its effective amount in case the compound of this invention is not administered. In this way, undesired side effects associated with high doses of either agent may be minimized. Other potential advantages (including without limitation improved dosing regimens and/or reduced drug cost) will be apparent to those skilled in the art.

In various embodiments, the therapies (e.g., prophylactic or therapeutic agents) are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In preferred embodiments, two or more therapies are administered within the same patient's visit.

In certain embodiments, one or more of the rifamycin class antibiotic (e.g., rifaximin) and one or more other therapies (e.g., prophylactic or therapeutic agents) are cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time, optionally, followed by the administration of a third therapy (e.g., prophylactic or therapeutic agent) for a period of time and so forth, and repeating this sequential administration, i.e., the cycle in order to reduce the development of resistance to one of the therapies, to avoid or reduce the side effects of one of the therapies, and/or to improve the efficacy of the therapies.

In certain embodiments, the administration of the same compounds may be repeated and the administrations may be separated by at least about 1 day, 2 days, 3 days, 5 days, 10 days, 15 days, 30 days, 45 days, 3 weeks, 4 weeks, 5 weeks, 6 weeks, 12 weeks, 2 months, 75 days, 3 months, or at least 6 months. In other embodiments, the administration of the same therapy (e.g., prophylactic or therapeutic agent) other than a rifamycin class antibiotic (e.g., rifaximin) may be repeated and the administration may be separated by at least at least 1 day, 2 days, 3 days, 5 days, 10 days, 15 days, 30 days, 45 days, 2 months, 75 days, 3 months, or at least 6 months. In one embodiment, a label on a rifamycin class antibiotic may instruct, for example, do not repeat more often than every 6 weeks. In another embodiment, a label on a rifamycin class antibiotic may instruct, for example, do not repeat more often than every 3 weeks. In another embodiment, a label on a rifamycin class antibiotic may instruct, for example, do not repeat more often than every 3 - 12 weeks. Included within ranges given herein for dosage or administration are any value within the range.

In certain embodiments, retreatment is efficacious in combination with the methods disclosed herein. See for example, Rifaximin versus Other Antibiotics in the Primary Treatment and Retreatment of Bacterial Overgrowth in IBS, Janet Yang, Hyo-Rang Lee, Kimberly Low, Soumya Chatterjee, and Mark Pimentel, Dig Dis Sci (2008) 53:169-174. For example, methods as described herein may further comprise determining symptom relief in a subject and administering a second course of rifaximin treatment if symptoms remain unresolved. Methods may also further comprise, for example, determining the gender of a subject and administering the therapeutically effective amount to a male subject.

### Kits

Kits are also provided herein, for example, kits for treating an infection in a subject. The kits may contain, for example, a polymorph or amorphous form of rifaximin and instructions for use. The instructions for use may contain prescribing information, dosage information, storage information, and the like.

In one embodiment, the label describes a length of treatment with the rifamycin class antibiotic, whereby a subject is removed from treatment if a healthcare professional prescribes the rifamycin class antibiotic according to the label instructions.

Label instructions, include, for example, instructions to take the rifamycin class antibiotic for 14 days for the treatment of IBS. The instructions could also read, for example, take for 1650 mg/day of rifaximin for 14 days for acute treatment or prevention of an infection.

Packaged compositions are also provided, and may comprise a therapeutically effective amount of one or more of a one or more of an amorphous form, Form α, Form β, Form γ, Form δ, Form ε, Form ζ, or Form η polymorph of rifaximin of rifaximin and a pharmaceutically acceptable carrier or diluent, wherein the composition is formulated for treating a subject suffering from or susceptible to a an infection, and packaged with instructions to treat a subject suffering from or susceptible to an infection.

### EXAMPLES

It should be appreciated that the invention should not be construed to be limited to the example, which is now described; rather, the invention should be construed to include any and all applications provided herein and all equivalent variations within the skill of the ordinary artisan.

### Example 1: Rifaximin Alters Bacterial Attachment

The data presented in this experiment demonstrate that rifaximin mediated changes to various epithelial cell types reduced E. coli, e.g., EAEC, attachment by biologically altering the host cell. These effects extend to *B. anthracis* adherence and internalization. These observations demonstrate that significant functional differences between rifaximin and its cousin rifampin exist.

### MATERIALS AND METHODS

**Bacterial strains and cell lines.** EAEC strain 042 (provided by J. Nataro, University of Maryland School of Medicine, Baltimore, MD) (20), S. sonnei (a clinical isolate obtained from a patient in India by our laboratory in 2008), and B. anthracis Sterne strain 7702 (provided by T. M. Koehler, University of Texas Health Science Center, Houston, TX) were used in this study. HEp-2 (a human larynx squamous cell carcinoma) (5, 12), HCT-8 (a human intestinal adenocarcinoma cell line) (11), A549 (a human lung adenocarcinoma epithelial cell line) (24), and HeLa (a cervical cancer cell line) were obtained from the American Type Culture Collection. These cell lines were used in this study and were maintained in Dulbecco's minimal essential medium (DMEM) containing 10% fetal bovine serum (FBS) in a humidified incubator with 5% CO2. No antibiotics were used in the preparation of the medium.

**HEp-2 cell assay.** The HEp-2 cell attachment assay was carried out as described previously (11), except that confluent monolayers were used instead of cells grown to 50 to 80% confluence unless otherwise specified. HEp-2 cells were grown in Lab-Tek II (Nunc, Rochester, NY) chambered slides and were incubated in the presence of antibiotics or the appropriate controls for 4, 8, 16, 18, and 24 h. At the end of the antibiotic incubation period, chambers were washed three times with PBS (phosphate-buffered saline, pH 7.4) prior to the addition of EAEC (7 x 10⁶ to 1 x 10⁷ bacteria in 1 ml DMEM, 10% FBS, and 1% D-mannose) (2, 9) that had been cultured at 37°C overnight in Trypticase soy broth (Difco, Lawrence, KS) with 1% D-mannose (Sigma, St. Louis, MO). After a 3-h incubation at 37°C, the attachment of E. coli 042 to epithelial cells was visualized microscopically following Wright-Giemsa staining (Protocol Hema 3; Fisher Diagnostics, Middletown, VA) using an Olympus BX60 inverted microscope attached to an Olympus C-5060 digital camera.

**Quantification of bacterial adherence and internalization.** HEp-2, HCT-8, A459, or HeLa cells were grown to confluence in 24-well plates (Corning Inc., Corning, NY) and incubated with the respective antibiotics or controls. Twenty four hours later, the supernatants were removed, and the wells were washed gently three times with 1 ml PBS prior to the addition of either EAEC strain 042 (2, 9) or S. sonnei (7 x 10⁶ to 1 x 10⁷ in 1 ml DMEM, 10% FBS, 1% D-mannose) grown at 37°C overnight in Trypticase soy broth with 1% D-mannose, or B. anthracis spores (4.8 x 10⁵) prepared from Sterne strain 7702 in phage assay (PA) medium as described previously (10, 24, 25).

For the quantification of bacterial adherence, EAEC 042 was incubated in the presence of the respective antibiotic-pretreated cell lines, and S. sonnei was incubated in the presence of antibiotic-pretreated HeLa cells, in 24-well plates for 3 h at 37°C. Following this incubation, the supernatants were removed, and the wells were washed three times with 1 ml sterile PBS. Distilled water (1 ml) was then added to the wells, and the cells were removed by pipetting, diluted in sterile PBS, and plated on Luria-Bertani (LB) agar plates for quantification by counting of CFU 16 h later. For B. anthracis attachment, A549 cells were grown to confluence in 24-well tissue culture plates and pretreated for 24 h with antibiotics as described above. The cells were washed three times with PBS to remove antibiotics and were then infected with Sterne strain 7702 spores for 1 h at 37°C in a humidified chamber with 5% CO2. Unbound spores were removed by three washes with PBS, and the A549 cells were removed and plated on LB agar plates as described above for CFU determinations.

For the internalization assays, S. sonnei or B. anthracis was incubated with antibiotic-treated HeLa or A549 cells, respectively, as described above. After the bacteria were incubated with the respective cell lines and the wells were washed, the epithelial cells were further incubated with a medium containing gentamicin (100 µg/ml) for 1 h to kill noninternalized bacteria, washed with PBS, lysed in distilled water, and plated for quantification as described above.

In one experiment, the number of EAEC bacteria in the supernatants following the 3-h incubation with HEp-2 cells treated with various antibiotics or controls was determined. Supernatants were collected, and the wells were washed three times with 200 µl sterile PBS. The PBS washes and the supernatants were combined, centrifuged, and resuspended in 1 ml sterile PBS; the suspension was then serially diluted and plated on LB agar plates for quantification as described above.

The CFU data are means ± standard errors (SE) from triplicate wells for bacteria that adhered or were internalized and are expressed as percentages of the total bacteria added to the respective treatment wells. Statistical differences in bacterial counts were determined using Student's t test.

**Antibiotics.** Rifaximin (Salix Pharmaceuticals Inc., Morrisville, NC), rifampin (Sigma), doxycycline (Sigma), or gentamicin (Sigma) were tested in attachment/ internalization assays. Rifaximin was dissolved in acetone and rifampin, and doxycycline and gentamicin were dissolved in water (1-µg/µl stocks of each were prepared). Antibiotics were prepared fresh at the start of each experiment. Antibiotics and acetone were sterile filtered with a 0.22-µM-pore-size syringe filter before use. Antibiotics were incubated with HEp-2 cells for various times at 8, 32, or 64 µg/ml and with HCT-8, A549, or HeLa cells for 24 h at 32 or 64 µg/ml. Acetone was administered at a volume equivalent to the volume used to deliver each respective antibiotic concentration as a negative control. The concentrations tested were based on the MICs of rifaximin and rifampin. The highest dose of rifaximin selected (64 µg/ml) corresponded to the MIC50 previously established for EAEC in our laboratory.

**Supernatant dialysis.** HEp-2 cells were cultured in T-175 flasks as described above. When the cells reached confluence, the medium was removed and replaced with fresh medium containing either no antibiotics, rifaximin, rifampin, doxycycline, or acetone (64 µg/ml or acetone at the same volume used to deliver the antibiotics). After 24 h, the medium was collected, loaded into regenerated cellulose dialysis tubing (cutoff, 6 to 8 kDa; Spectra/Por, Rancho Domiguez, CA), and dialyzed against 4 liters of PBS changed four times. Each dialysis step lasted at least 6 h and was carried out at 4°C. Dialyzed supernatants were either used immediately in adherence assays or stored at -20°C until use.

The effect of supernatants on EAEC adherence was determined by mixing EAEC with the dialyzed media from the different antibiotic (or control) treatment groups and adding the bacteria to HEp-2 cells cultured in chambered slides or to chambered slides with no HEp-2 cells. Adherence was visualized microscopically as described above.

**Cytokine arrays.** HEp-2 cells were plated on chambered slides and grown to confluence. The medium was replaced with fresh medium containing either antibiotics (64 µg/ml), a diluent control (64 µl) (acetone), or medium with no antibiotics. Supernatants were collected 24 h later and were used to probe a cytokine array membrane (Panomics, Fremont, CA) that supported the detection of Apo/Fas, cytotoxic T-lymphocyte-associated antigen (CTLA), eotaxin, granulocyte-monocyte colony-stimulating factor (GM-CSF), epidermal growth factor (EGF), gamma interferon (IFN-γ)-inducible protein 10 (IP-10), leptin, monocyte inflammatory protein 1α (MIP1α), MIP1, MIP4, MIP5, matrix metalloprotease 3 (MMP3), RANTES (regulated on activation, normal T-expressed and secreted), transforming growth factor β (TGF-β), IFN-γ, tumor necrosis factor alpha (TNF-a), TNF receptor I (TNFRI), TNFRII, intracellular adhesion molecule 1 (ICAM-1), vascular cell adhesion molecule 1 (VCAM-1), vascular endothelial growth factor (VEGF), interleukin-la (IL-1α), IL-1, IL-Rα, IL-2, IL-3, IL-4, IL-5, IL-6, IL-6R, IL-7, IL-8, IL-10, IL-12 (p40), IL-15, and IL-17 as described by the manufacturer. The presence of the respective supernatant proteins was visualized by exposing and then developing Amersham Hyperfilm ECL (GE Healthcare, Buckinghamshire, United Kingdom) film. Blots probed with the respective supernatants were analyzed at the same time and developed simultaneously on the same Hyperfilm.

### RESULTS

**Effect of rifaximin on EAEC adherence.** The effect of rifaximin on bacterial attachment was first examined using the standard HEp-2 cell adherence assay commonly carried out to phenotypically define EAEC isolates based on their stackedbrick adherence pattern (8, 15). HEp-2 cells were grown to confluence and were then incubated with rifaximin, rifampin, doxycycline, or acetone. After 24 h, the wells were washed prior to the addition of the EAEC strain 042. EAEC 042 adhered in the traditional stacked-brick formation to untreated HEp-2 cells (Fig. 1A) or HEp-2 cells treated with rifampin, doxycycline, or acetone (data not shown); however, the level of adherence to rifaximin-pretreated HEp-2 cells was greatly reduced (Fig. 1B). These effects were time and concentration dependent; however, since pretreatment with 64 mg/ml rifaximin for 24 h had the greatest effects on bacterial attachment, this dose was used in subsequent experiments.

EAEC adherence to HEp-2 cells following pretreatment with the various antibiotics or controls was quantified. The level of EAEC adherence to rifaximin-pretreated cells was significantly lower than the level of adherence to untreated cells or to cells treated with either rifampin, acetone (Fig. 1C), or doxycycline. No differences in bacterial counts were observed in the supernatants (Fig. 1C).

To determine if the observed reduction in EAEC adherence following rifaximin pretreatment of HEp-2 cells extended to other cell types or bacteria, we examined EAEC 042 adherence to three additional cell lines. We examined the effects of rifaximin pretreatment on adherence and internalization using two vastly distinct pathogens: B. anthracis and S. sonnei.

EAEC adherence to HEp-2, HCT-8, A549, and HeLa cells was measured by carrying out adherence assays as described above. The respective cell lines were grown to confluence in 24-well plates, and fresh medium containing no antibiotics or 64 mg of either rifaximin, rifampin, doxycycline, or acetone (64 ml) was added for 24 h. Rifaximin pretreatment significantly reduced EAEC adherence to HEp-2, HeLa, and A549 cells. Slightly fewer bacteria adhered to rifaximin-pretreated HCT-8 cells, but this difference was not significant, suggesting differences in cellular susceptibility to rifaximin (Fig. 2).

The adherence and internalization of B. anthracis and S. sonnei was measured to determine if rifaximin pretreatment affected these processes in different bacteria. B. anthracis is a Gram-positive spore-forming bacteria that is the causative agent of anthrax. Humans become infected via spore entry into the lungs or gut or via skin abrasions; infections can result in sepsis and secondary manifestations, including meningitis and potentially death. Recently, the laboratory of Y. Xu established a model of adherence and internalization (using the gentamicin protection assay) for B. anthracis Sterne strain 7702 in A549 lung epithelial cells that was modified in this study to examine the role of rifaximin in these processes. S. sonnei is a Gram-negative member of the family Enterobacteriaceae that causes disease in humans by invading and replicating in cells lining the colonic mucosa. Of the four Shigella species, S. sonnei is the most common cause of shigellosis in developed countries.

Following incubations with the respective antibiotics (or controls), significant reductions in both the adherence and the internalization of B. anthracis were observed in rifaximin-pretreated A549 cells compared to those for the other treatment groups, including an internal gentamicin-only treatment group (Fig. 3A and B). In contrast, rifaximin pretreatment of HeLa cells had no effect on S. sonnei attachment or internalization compared to those for the other treatment groups under the conditions examined (Fig. 3C and D).

**Effect of HEp-2 supernatants on EAEC adherence.** Because EAEC adherence to HEp-2 cells in the stacked-brick formation is the gold standard for defining EAEC isolates, we next attempted to determine if the observed reduction in EAEC adherence following rifaximin pretreatment was due to rifaximin-mediated alterations in HEp-2 cell physiology that could be conferred via the supernatants of treated cells. To this end, HEp-2 cells were grown in a medium containing 64 mg/ml of either rifaximin, rifampin, or doxycycline (or acetone [64 µl/ml]) for 24 h. Supernatants were then collected and dialyzed extensively using 6- to 8-kDa-cutoff dialysis tubing to remove any trace of the antibiotics used in the respective treatment groups. This ensured that when the dialyzed media corresponding to the respective antibiotic treatment groups were mixed with EAEC in order to examine the effects of the supernatants on adherence, any changes observed would not be due to antibiotics in the medium. After dialysis, the supernatants were sterile filtered and mixed with EAEC, and adherence to chambered slides with or without HEp-2 cells (50% confluent) was examined. After a 3-h incubation, the slides were washed and stained as described above. EAEC that was mixed with dialyzed supernatants from HEp-2 cells only, i.e., with no antibiotic treatment (Fig. 4A and C), or with dialyzed supernatants from HEp-2 cells pretreated with either rifampin (Fig. 4B), doxycycline, or acetone, adhered to chambers independently of the presence of HEp-2 cells (Fig. 4A and B, with HEp-2 cells; Fig. 4C, without HEp-2 cells). EAEC mixed with PBS and added directly to chambers for 3 h adhered in a fashion indistinguishable from the adherence pattern visible in Fig. 4A to C (data not shown), suggesting that the bacterial adherence pattern was not affected by the medium/buffer used in the adherence assay. In contrast, EAEC that was mixed with dialyzed supernatants from rifaximin-treated HEp-2 cells adhered poorly to chambered wells in the presence or absence of HEp-2 cells (Fig. 4D and E, respectively).

**Cytokine arrays.** Since the supernatants collected from rifaximin- treated HEp-2 cells had the capacity to confer changes in EAEC adherence, we defined the cytokine profiles of the respective supernatants incubated for 24 h alone (Fig. 5A) or pretreated (24 h) with either rifampin (Fig. 5B) or rifaximin (Fig. 4C). GM-CSF, MIP4, MIP5, MMP3, RANTES, TGF-b, IFN-g, TNFRI, TNFRII, VCAM-1, VEGF, IL-4, IL-6, IL-8, IL-12 (p40), and IL-15 could be found in the supernatants of untreated (Fig. 5A), doxycycline-treated, or acetone-treated (data not shown) cells. Rifampin-treated cell supernatants had the same profile, except that MIP5 was not detected (Fig. 5B). In contrast, supernatants analyzed from HEp-2 cells cultured in the presence of rifaximin contained detectable levels of RANTES and IL-4 only (Fig. 5C).

### DISCUSSION

In this example the inventors demonstrated that rifaximin pretreatment decreased bacterial adherence to HEp-2 cells without affecting EAEC viability, since equal numbers of bacteria were recoverable from the supernatants of all treatment groups. In contrast, the number of bacteria that adhered to confluent HEp-2 cells pretreated with rifaximin was significantly lower than the number of EAEC bacteria recoverable from cells treated with control antibiotics or left untreated.

The data presented in this example demonstrates that rifaximin mediated changes to various epithelial cell types reduced EAEC attachment by biologically altering the host cell. These effects extend to *B. anthracis* adherence and internalization. These observations demonstrate that significant functional differences between rifaximin and its cousin rifampin exist.

### Example 2: Rifaximin-Induced Alteration of Virulance of E. Coli and S. Sonnei

The following experiment demonstrates that rifaximin shortens the duration of travelers' diarrhea without important alteration of colonic flora. This study investigated the expression of virulence factors (heat-stable [ST] and heat-labile [LT] enterotoxins, surface adhesion factors [CS2/CS3, CS6], and matrix metalloproteinase-9 [MMP-9]) and the interleukin-8 (IL-8) induction potential of diarrheagenic *Escherichia coli* and *Shigella sonnei* strains exposed to rifaximin (8, 32, and 64 mg/L) for 4, 8, 18, and 24 hours. ETEC isolates did not express ST/LT, CS2/CS3, or CS6; EAEC and *S sonnei* isolates did not produce detectable amounts of MMP-9. Induction of IL-8 was undetectable. At subinhibitory concentrations, rifaximin alters the virulence of ETEC, EAEC, and *S sonnei* isolates. These findings help explain the efficacy of rifaximin despite minimal alteration of colonic flora.

### Materials and methods

### Study isolates

Five enterotoxigenic *E coli* strains, 2 enteroaggregative *E coli* strains, and 1 S *sonnei* isolate were included in the study. The isolates had been identified previously from patients with TD acquired in Guadalajara, Mexico, during the summer of 2004 (5). Prior to any experimental procedures (Fig. 6), all study isolates were tested for minimum inhibitory concentration (MIC) against rifaximin. The susceptibility of all isolates to rifaximin was determined by agar dilution methodology following the recommendations of the Clinical and Laboratory Standards Institute. Mueller-Hinton (MH) broth was prepared according to the manufacturer's directions (Becton Dickinson, Cockeysville, MD), and rifaximin was dissolved in acetone and 2-fold dilutions prepared in MH broth.

### Exposure of isolates to rifaximin

All isolates were grown at 37°C in MH broth containing either acetone alone (for quality control) or varying concentrations of rifaximin (8, 32, or 64 mg/L) diluted in acetone for variable time periods (4, 8, 18, and 24 hours). MH broth was then plated onto MacConkey agar for growth. Viable ETEC isolates were then grown on colonization factor antigen agar (1% casamino acid, 0.15% yeast extract, 0.41 mM MgSO₄, 0.04 mM MnCl₂, 1% agarose, pH 7.4) plates for expression of ETEC colonization factors.

### RNA extraction and reverse transcriptase polymerase chain reaction (RT-PCR)

Total RNA was extracted from ETEC strains growing on MacConkey agar using an RNeasy Kit (Qiagen, Hilden, Germany). Extracted RNA was treated with DNase (DNase Amp grade kit; Invitrogen, Carlsbad, CA) according to manufacturer instructions. RNA concentration and integrity were determined using a spectrophotometer at 260/280 nm and by gel electrophoresis on 1% agarose gels. Samples were diluted and stored at -70°C until use.

All extracted RNA samples were subjected to 2-step RT-PCR. Reverse transcription was performed using the Sensiscript RT-PCR kit (Qiagen, Hilden, Germany). In summary, equal volumes (1.5 µL) of total RNA (1.5 pg - 15 ng) were mixed in 10 µL reaction buffer (IX reverse transcriptase [RT] buffer, 200 nM dNTP, 1 unit of RT, 5 units of RNase Inhibitor [Roche, Mannheim, Germany]) with 800 nM reverse primers for heat-stable (ST) and heat-labile (LT) enterotoxins and the surface adhesion factors CS2/CS3 and CS6 and incubated at 37°C for 1 hour. Samples were then heated to 93°C for 5 minutes to inactivate the RT enzyme.

The RT reactions were followed by PCR amplification for ST, LT, CS2/CS3, and CS6 genes. The polymerase chain reaction was performed in 50-µL reactions containing 1 unit *Taq* polymerase (Sigma-Aldrich, St Louis, MO), 5-µL 10X PCR reaction buffer (1.5 mM MgCl₂ [Sigma-Aldrich, St Louis, MO], 200 nM dNTP [Roche, Mannheim, Germany]), and 80 nM of each primer. The following primer pairs were used: ST, forward 5'-tcaccttcccctcaggatg-3' and reverse 5'-tacaagcaggattacaacac-3'; LT, forward 5'-acggcgttactatcctctc-3' and reverse 5'-tggtctcggtcagatatgtg-3'; CS2/CS3, forward 5'-agccacactggaactgagac-3' and reverse 5'-agccatttcaccgctacac-3'; CS6, forward 5'-ctttgaggggcaggttttcgt-3' and reverse 5'- cttagagagaggccgtcgga-3'. Amplification conditions involved an initial denaturation step at 94°C for 5 minutes followed by 25 cycles of 94°C (30s), 57°C (30s), and 72°C (30s). Final elongation was performed at 72°C for 5 minutes. Amplification of LT required slightly altered conditions, including an increase in MgCl₂ to 2 mM and an increase of each primer to 160 nM. Also, the number of cycles was increased to 27, and the annealing temperature was decreased to 55°C. Polymerase chain reaction products were visualized using a 1% agarose gel. Single-band PCR products of the correct sizes for ST, CS2/CS3, and CS6 genes were obtained in all reactions.

### Matrix metalloproteinase-9 detection

Expression of matrix metalloproteinase-9 (MMP-9) was analyzed using gelatin zymography in all viable EAEC and *S. sonnei* isolates growing on MacConkey agar. Isolates were suspended in 1 mL of 0.1 M phosphate buffered saline (pH 7.2) and centrifuged at 1000*g* for 10 minutes at 4°C. The supernatant was subjected to gelatin zymography to detect MMP-9 activity using a modified Heussen and Dowdle protocol (9). In summary, the supernatant was diluted 1:2 in a sample buffer (125 mM Tris-HCl, pH 6.8, 20% glycerol, 4% sodium dodecyl sulfate, 0.02% bromphenol blue), and 20 µL was loaded on to a 10% SDS-PAGE gel containing 1 mg/mL gelatin. Prestained standard proteins were used as molecular weight markers. After electrophoresis, gels were rinsed with incubation buffer (2.5% triton X-100, 50 mM Tris-HCl, pH 7.4, 0.02% Brij 35, 10 mM CaCl₂, 2 µM ZnCl₂) for 1 hour at room temperature to remove SDS. Gels were then incubated overnight at 37°C and subsequently stained with Coomassie brilliant blue R-250 (Thermo Fisher Scientific, Inc, Rockford, IL). Proteins possessing gelatinolytic activity appeared as clear lytic bands in the background. Matrix metalloproteinase-9 was identified among all gelatinolytic proteins by size.

### HCT-8 cell culture assay for interleukin-8 (IL-8) release

The human intestinal adenocarcinoma cell line, HCT-8, was used to study inflammatory modulators as previously published (Huang, 2004 #15). Monolayers of HCT-8 cells (ATCC CCL-244; American Type Culture Collection, Rockville, MD) were grown in 24-well culture plates (Corning Costar, Corning, NY) in an RPMI 1640 medium (Sigma-Aldrich Co, St Louis, MO) supplemented with 100 U/mL of penicillin, 100 µg/mL of streptomycin, and 0.25 µg/mL of amphotericin B. They were maintained in a medium supplemented with 10% fetal bovine serum (HyClone, Logan, UT) and grown until approximately 4 x 10⁵ cells/well confluence.

HCT-8 cells were infected with EAEC strains recovered from MacConkey agar in triplicate at a 100:1 ratio in an antibiotic- and serum-free RPMI 1640 medium. Cells were incubated for 3 hours in a humidified chamber at 37°C in an atmosphere containing 5% CO₂, after which supernatants were collected. The supernatant concentration of IL-8 in each well was determined by using a commercially available enzyme-linked immunosorbent assay kit for human IL-8 (R&D Systems, Minneapolis, MN). All samples were tested twice, and the average value was reported.

### Results

### MIC values

Antimicrobial susceptibility testing of all isolates was performed prior to initiation of any experimental procedures. All ETEC and EAEC isolates had MIC values ranging from 64 mg/L to 128 mg/L (3 ETEC isolates had 64mg/L and 2 had 128mg/L; 2 of EAEC isolates had MIC against rifaximin 64mg/L), whereas the *S sonnei* isolate had an MIC value of 16 mg/L.

### Isolate viability after rifaximin exposure

All ETEC strains were viable after exposure to rifaximin 8, 32, and 64 mg/L for 4 and 8 hours and after exposure to rifaximin 8 and 32 mg/L for 18 hours. No ETEC isolates were viable after exposure to rifaximin 64 mg/L for 18 hours or rifaximin 32 and 64 mg/L for 24 hours. EAEC isolates were viable at 4, 8, and 18 hours for rifaximin 8, 32, and 64 mg/L. The EAEC isolates were also viable after exposure to rifaximin 8 mg/L for 24 hours, although no viable isolates were recovered after exposure to rifaximin 32 and 64 mg/L for 24 hours. The *S sonnei* isolate was viable when exposed to rifaximin 8 mg/L for 4, 8, 18, and 24 hours; however, it was not viable after exposure to rifaximin 32 or 64 mg/L at any time point examined.

### Virulence factors in ETEC isolates

Expression of virulence factors by ETEC isolates exposed to rifaximin is summarized in Table 1. Viable ETEC isolates expressed all examined virulence factors (ST, LT, CS2/CS3, CS6) after 4- and 8-hour exposure to rifaximin 8, 32, and 64 mg/L. At the 18-hour time point, all virulence factors were expressed after exposure to rifaximin 8 mg/L, but no factors were seen after exposure to rifaximin 32 mg/L. Isolates were not viable when exposed to rifaximin 64 mg/L for 18 hours. Although isolates were viable after 24-hour exposure to rifaximin 8 mg/L, these isolates did not express any virulence factors. Isolates were not viable after exposure to rifaximin 32 or 64 mg/L at the 24-hour time point.

**Table 1. Expression of virulence factors in ETEC isolates after exposure to rifaximin**

| **Virulence factor** | **Exposure time, (hrs)** | **Rifaximin concentration,^{a} mg/L** | | |
|---|---|---|---|---|
| | | **8** | **32** | **64** |
| ST | | + | + | + |
| LT | | + | + | + |
| ST/LT | 4 | + | + | + |
| CS2/CS3 | | + | + | + |
| CS6 | | + | + | + |
| ST | | + | + | + |
| LT | | + | + | + |
| ST/LT | 8 | + | + | + |
| CS2/CS3 | | + | + | + |
| CS6 | | + | + | + |
| ST | | + | - | NV |
| LT | | + | - | NV |
| ST/LT | 18 | + | - | NV |
| CS2/CS3 | | + | - | NV |
| CS6 | | + | - | NV |
| ST | 24 | - | NV | NV |
| LT | | - | NV | NV |
| ST/LT | | - | NV | NV |
| CS2/CS3 | | - | NV | NV |
| CS6 | | - | NV | NV |

| | | | | |
|---|---|---|---|---|
| CS, coli surface antigen (adhesion); ETEC, enterotoxigenic *E coli;* LT, heat-labile toxin; NV, not viable; ST, heat-stable toxin; ST/LT, heat-stable and heat-labile toxin. ^{a} + = present; - = not present. | | | | |

### Virulence factors in EAEC and S sonnei isolates

The EAEC isolates expressed MMP-9 after exposure to rifaximin 8 mg/L for 4 and 8 hours. At the 18-hour time point, one EAEC isolate produced MMP-9, but the other isolate did not. Both isolates were viable after rifaximin 8 mg/L for 24 hours, but MMP-9 was undetectable. Matrix metalloproteinase-9 was also undetectable in the supernatants of either isolates after rifaximin 32 or 64 mg/L for 4, 8, or 18 hours, although they were viable at these time points. Both isolates were not viable after rifaximin 32 or 64 mg/L at the 24-hour time point. In the *S sonnei* isolate, MMP-9 was detectable only after exposure to rifaximin 8 mg/L for 4 hours; at other time points and rifaximin concentrations, MMP-9 was either undetectable or the isolate was not viable.

EAEC strains exposed to rifaximin 8 or 32 mg/L for 4 or 8 hours elicited production of IL-8 from HCT-8 cells. The EAEC isolates exposed to rifaximin 64 mg/L for 4 hours also caused IL-8 production. Interleukin-8 was either undetected following HCT-8 infection with EAEC strains, or EAEC strains were not viable at all other time points and rifaximin concentrations.

This example demonstrated that subinhibitory concentrations of rifaximin (ie, concentrations that allowed viability) altered the virulence and biologic properties of the two principal causes of travelers' diarrhea (ETEC and EAEC) and *S sonnei.* Expression of ETEC virulence factors ST, LT, CS2/CS3, and CS6 was reduced upon exposure to subinhibitory concentrations of rifaximin. Viable EAEC isolates that were exposed to rifaximin 8, 32, or 64 mg/L for 18 hours or rifaximin 8 mg/L for 24 hours did not elicit IL-8 production from HCT-8 cells in vitro. In addition, MMP-9 production in EAEC or *S sonnei* was inhibited upon rifaximin exposure. Taken together, these data suggest a loss of virulence factors in pathogens isolated from patients with TD following in vitro exposure to subinhibitory concentrations of rifaximin.

This study provides an explanation for the finding that the duration of TD due to diarrheagenic *E coli* is successfully shortened with rifaximin treatment without major reduction in counts of pathogens and colonic flora. In the treatment of noninvasive bacterial GI infection, rifaximin may have an initial effect on organisms' virulence followed by concentration- and time-dependent inhibition of growth and pathogen death. The multiple beneficial qualities of rifaximin make it an ideal antibiotic for the prevention and treatment of TD.

### Example 3: In Vitro Susceptibility of C. difficile to Rifaximin and Rifampin

This example demonstrates that rifaximin has high-level activity against *C difficile i*n vitro. Determination of resistance to rifampin did not predict rifaximin resistance.

### METHODS

### BACTERIAL STRAINS

Stool samples were collected from consecutive adult patients who developed diarrhea between August 2006 and December 2007 while hospitalized in a university hospital in the Texas Medical Center in Houston. All stool samples were screened for *C difficile* cytotoxin B via tissue culture assay (Tech Lab® Inc, Blacksburg, VA) by the hospital clinical laboratory. Samples positive for cytotoxin B were submitted to the research laboratory within 3 days of collection, and approximately 1 g or 1 mL of feces was gently mixed with an equal volume of absolute ethanol and incubated at room temperature for 60 minutes. The supernatant was discarded, and an aliquot of the resulting pellet was inoculated onto *C difficile*-selective cycloserine-cefoxitin fructose agar plates (Remel, Lenexa, KS). The plates were cultured under anaerobic conditions at 37°C for 48 to 72 hours. Putative colonies of *C3 difficile* were identified by morphology and odor and then stored at -80°C in Trypticase™ soy broth (Becton, Dickinson and Company, Sparks, MD) with 7% horse blood. *C difficile* colonies were confirmed by polymerase chain reaction (PCR) of a coding region of the 16S rRNA gene.[26] Confirmed C *difficile* isolates and two control strains (ATCC 43593 and ATCC 700057) were analyzed by PCR for the presence of genes for toxins A and B and BT and for *tcdC*-del.

### SUSCEPTIBILITY TESTING

Susceptibility of *C difficile* isolates to rifampin was determined with rifampin E strips (Etest®; AB Biodisk, Piscataway, NJ). Susceptibility of *C difficile* strains to rifaximin (Xifaxan®; Salix Pharmaceuticals, Inc, Morrisville, NC) was determined with agar dilution methodology based on standards developed by the Clinical and Laboratory Standards Institute® (CLSI).[29] Rifaximin was dissolved in acetone at a concentration of 10,240 µg/mL, and sequential two-fold dilutions were made in sterilised water to provide concentrations ranging from 1024 µg/mL to 0.016 µg/mL. Diluted rifaximin was added to Mueller Hinton (MH) agar prepared according to the manufacturer's instructions (Becton, Dickinson and Company, Sparks, MD). *C difficile* isolates were inoculated onto the MH agar plates and incubated at 37°C for 48 hours under anaerobic conditions. For quality control, the minimal inhibitory concentrations (MICs) for control strains of *Escherichia coli* (ATCC 25922), *Staphylococcus aureus* (ATCC 29213), and *C difficile* (ATCC 700057) were determined for each antimicrobial agent. The lowest concentration of test antimicrobial agent that completely inhibited visible growth of the strain on the agar dilution plate was the MIC. Resistance to rifaximin or rifampin was defined as MIC ≥32 µg/mL.

### RESULTS

A total of 396 bacterial isolates resembling *C difficile* obtained from 324 patients who developed diarrhea after being hospitalised were analyzed. Of the 396 isolates analyzed, 359 were confirmed as *C difficile.* Rifaximin and rifampin exhibited high levels of activity against *C difficile* isolates, with an MIC at which 90% of isolates were inhibited (MIC₉₀) of 0.25 µg/mL and 4 µg/mL, respectively; the MICs at which 50% of isolates were inhibited (MIC₅₀) were <0.01 µg/mL and <0.002 µg/mL, respectively (table 2). Twenty-eight of the 359 isolates analyzed (8%) were resistant to rifampin, and six of these 28 isolates were resistant to rifaximin (ie, MIC value ≥32 µg/mL; table 3). A total of 11 of the 359 (3%) isolates were resistant to rifaximin alone. All control isolates had MIC values within the established ranges published by the CLSI.[29]

**Table 2. Minimum inhibitory concentrations for rifaximin and rifampin against C difficile**

| | | MIC (µg/mL) | | |
|---|---|---|---|---|
| Antibiotic | Isolates (N) | MIC₅₀ | MIC₉₀ | Range |
| Rifaximin | 359 | <0.01 | 0.25 | <0.01 to >1024 |
| Rifampin | 359 | <0.002 | 4 | <0.002 to >32 |

**Table 3. Distribution of isolates by MIC value**

| Antibiotic | MIC (µg/mL) | | | | | Isolates (N) |
|---|---|---|---|---|---|---|
| | ≤0.003 | -0.25 | -2 | -24 | ≥32 | |
| Rifaximin, n | 302 | 24 | 16 | 6 | 11 | 359 |
| Rifampin, n | 273 | 27 | 18 | 13 | 28 | 359 |

Of the 359 *C difficile* isolates analyzed, 318 (89%) contained genes for toxins A and B, 106 (30%) contained the gene encoding BT, and 86 (24%) contained *tcdC-del.* Fifty-five (15%) isolates were positive for BT and *tcdC-del.* Rifampin- and rifaximin-resistant isolates differed in their overall genetic composition (table 4).

**Table 4. Genetic composition of C difficile isolates resistant to rifampin or rifaximin^{a}**

| Genetic characterisation | | | | Isolates resistant to rifaximin alone (n=11), n (%) | Isolates resistant to rifampin (n=28), n (%) |
|---|---|---|---|---|---|
| A | B | BT | *tcdC*-del*^{a}* | | |
| + | + | + | + | 0 (0) | 2 (7) |
| + | + | - | + | 1 (9) | 5 (18) |
| + | + | + | - | 2 (18) | 4 (14) |
| + | + | - | - | 6 (55) | 13 (46) |
| - | - | - | - | 2 (18) | 4 (14) |

| | | | | | |
|---|---|---|---|---|---|
| *^{a} tcdC*-del, partial *tcdC* gene deletions. | | | | | |

### DISCUSSION

In the current in vitro study, rifaximin demonstrated high levels of activity against *C difficile* isolates. The low MIC values observed for rifaximin against *C difficile* isolates in this study (MIC₅₀ of <0.01 µg/mL; MIC₉₀ of 0.25 µg/mL) resemble data from previous studies. An analysis of 110 *C difficile* isolates obtained from the United States (including recent epidemic strains), South America, and Europe between 1983 and 2004 reported MIC₅₀ and MIC₉₀ values for rifaximin of 0.0075 µg/mL and 0.015 µg/mL, respectively.[21] Similarly, an examination of 201 *C difficile* isolates obtained from two tertiary care facilities and a public health laboratory in Canada between 1992 and 2007 showed MIC₅₀ and MIC₉₀ values for rifaximin of 0.008 µg/mL and 0.015 µg/mL, respectively (JA Karlowsky, N Laing, M Alfa, et al, presented at the 47th Annual Interscience Conference on Antimicrobial Agents and Chemotherapy, Chicago, IL, 17 to 20 September 2007). In the present study, the MIC₉₀ for rifaximin (0.25 µg/mL) was lower than that for rifampin (4 µg/mL), suggesting that rifaximin may be more effective than rifampin against CDI.

The present work also demonstrated that fewer isolates were resistant to rifaximin (11 of 359 isolates [3%]) than to rifampin (28 of 359 isolates [8%]), despite the fact that the percentage of rifampin-resistant isolates is lower in the present study than that previously reported.[30] The low percentage of isolates resistant to rifaximin demonstrated in this study is similar to that reported in other studies. In one study of 110 *C difficile* isolates obtained from multiple locations, 2.7% of the isolates demonstrated resistance to rifaximin, based on MIC values. In another study carried out in Canada, rifaximin had MIC values >2 µg/mL in only 4% of 201 *C difficile* isolates (JA Karlowsky, N Laing, M Alfa, et al, presented at the 47th Annual Interscience Conference on Antimicrobial Agents and Chemotherapy, Chicago, IL, 17 to 20 September 2007).

In the present work, only six of the 359 isolates (2%) were resistant to both rifampin and rifaximin, indicating a lack of correlation between rifampin and rifaximin resistance. This demonstrates that resistance to one rifamycin derivative does not predict resistance to all rifamycins.

### References

**1.** Jiang ZD, Lowe B, Verenkar MP, et al. Prevalence of enteric pathogens among international travelers with diarrhea acquired in Kenya (Mombasa), India (Goa), or Jamaica (Montego Bay). J Infect Dis 2002; 185:497-502.
2. DuPont HL, Jiang ZD, Ericsson CD, et al. Rifaximin versus ciprofloxacin for the treatment of traveler's diarrhea: a randomized, double-blind clinical trial. Clin Infect Dis 2001; 33:1807-15.
3. Gomi H, Jiang ZD, Adachi JA, et al. In vitro antimicrobial susceptibility testing of bacterial enteropathogens causing traveler's diarrhea in four geographic regions. Antimicrob Agents Chemother 2001; 45:212-6.
4. DuPont HL, Jiang ZD. Influence of rifaximin treatment on the susceptibility of intestinal Gram-negative flora and enterococci. Clin Microbiol Infect 2004; 10:1009-11.
5. DuPont HL, Jiang ZD, Okhuysen PC, et al. Antibacterial chemoprophylaxis in the prevention of traveler's diarrhea: evaluation of poorly absorbed oral rifaximin. Clin Infect Dis 2005; 41 Suppl 8:S571-6.
6. Jiang ZD, Ke S, Palazzini E, Riopel L, Dupont H. In vitro activity and fecal concentration of rifaximin after oral administration. Antimicrob Agents Chemother 2000; 44:2205-6.
7. Riva S, Fietta A, Berti M, Silvestri LG, Romero E. Relationships between curing of the F episome by rifampin and by acridine orange in Escherichia coli. Antimicrob Agents Chemother 1973; 3:456-62.
8. Clinical and Laboratory Standards Institute. Methods for Dilution Antimicrobial Susceptibility Tests for Bacterial that Grow Aerobically; Approved standard. CLSI. Wayne, PA, USA, 2006.
9. Heussen C, Dowdle EB. Electrophoretic analysis of plasminogen activators in polyacrylamide gels containing sodium dodecyl sulfate and copolymerized substrates. Anal Biochem 1980; 102:196-202.
10. Miyajima S, Akaike T, Matsumoto K, et al. Matrix metalloproteinases induction by pseudomonal virulence factors and inflammatory cytokines in vitro. Microb Pathog 2001; 31:271-81.
11. Huang DB, DuPont HL, Jiang ZD, Carlin L, Okhuysen PC. Interleukin-8 response in an intestinal HCT-8 cell line infected with enteroaggregative and enterotoxigenic Escherichia coli. Clin Diagn Lab Immunol 2004; 11:548-51.
12. Quiding-Jarbrink M, Smith DA, Bancroft GJ. Production of matrix metalloproteinases in response to mycobacterial infection. Infect Immun 2001; 69:5661-70.
13. Sorsa T, Tjaderhane L, Konttinen YT, et al. Matrix metalloproteinases: contribution to pathogenesis, diagnosis and treatment of periodontal inflammation. Ann Med 2006; 38:306-21.
14. Ripa S, Mignini F, Prenna M, Falcioni E. In vitro antibacterial activity of rifaximin against Clostridium difficile, Campylobacter jejuni and Yersinia spp. Drugs Exp Clin Res 1987; 13:483-8.
15. Taylor DN, Bourgeois AL, Ericsson CD, et al. A randomized, double-blind, multicenter study of rifaximin compared with placebo and with ciprofloxacin in the treatment of travelers' diarrhea. Am J Trop Med Hyg 2006; 74:1060-6.

### Incorporation by Reference

The contents of all references, patents, pending patent applications and published patents, cited throughout this application are hereby expressly incorporated by reference.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. Rifaximin for use in treating a rifampin resistant C. difficile infection (CDI), wherein the CDI is caused by a hypervirulant strain of C. difficile comprising genes encoding Toxin A, Toxin B, and Binary Toxin, and further comprising a deletion in the *tdc*C gene, and wherein the rifaximin is administered from 25 mg to 3000 mg per day.
